(19) Europäisches Patentamt
European Patent Office
Office européen des brevets

(11) **EP 2 348 995 B1**

(12) **EUROPEAN PATENT SPECIFICATION**

(45) Date of publication and mention
of the grant of the patent:
**07.11.2012 Bulletin 2012/45**

(51) Int Cl.:
*A61B 5/085* (2006.01)    *A61M 16/00* (2006.01)
*A61B 5/087* (2006.01)

(21) Application number: **09792853.5**

(22) Date of filing: **22.09.2009**

(86) International application number:
**PCT/US2009/057867**

(87) International publication number:
**WO 2010/036653 (01.04.2010 Gazette 2010/13)**

(54) **MODEL-PREDICTIVE ONLINE IDENTIFICATION OF PATIENT RESPIRATORY EFFORT DYNAMICS IN MEDICAL VENTILATORS**

MODELLVORHERSAGENDE ONLINE-IDENTIFIZIERUNG DER ATEMANSTRENGUNGSDYNAMIK VON PATIENTEN IN MEDIZINISCHEN BEATMUNGSGERÄTEN

IDENTIFICATION EN LIGNE A MODELE PREDICTIF D'UNE DYNAMIQUE D'EFFORT RESPIRATOIRE DE PATIENT DANS DES VENTILATEURS MEDICAUX

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **25.09.2008 US 238248**

(43) Date of publication of application:
**03.08.2011 Bulletin 2011/31**

(73) Proprietor: **Nellcor Puritan Bennett LLC Boulder, Colorado 80301 (US)**

(72) Inventor: **JAFARI, Mehdi Laguna Hills California 92653 (US)**

(74) Representative: **Beyer, Andreas Wuesthoff & Wuesthoff Patent- und Rechtsanwälte Schweigerstrasse 2 81541 München (DE)**

(56) References cited:
**WO-A-00/10634      WO-A-02/28460**

• **JAFARI M M ET AL: "Robust Feedback Design for Proportional Assist Ventilation-System Dynamics and Problem Definition" DECISION AND CONTROL, 2005 AND 2005 EUROPEAN CONTROL CONFERENCE. CDC-E CC '05. 44TH IEEE CONFERENCE ON SEVILLE, SPAIN 12-15 DEC. 2005, PISCATAWAY, NJ, USA,IEEE, 12 December 2005 (2005-12-12), pages 4839-4844, XP010884460 ISBN: 978-0-7803-9567-1**

**Description**

BACKGROUND

Embodiments of the present invention generally relate to mechanical ventilation, and more particularly to systems and methods for improving synchrony between patients and ventilators by using a computationally efficient model-predictive approach to determining patient respiratory effort using a clinically-based internal model of the patient muscle pressure generator.

Modem ventilators are designed to ventilate a patient's lungs with gas, and to thereby assist the patient when the patient's ability to breathe on their own is somehow impaired. A ventilated patient system consists of the patient's respiratory subsystem controlled by highly complex neural centers and physiologic feedback mechanisms, the ventilator's dynamics and delivery algorithms, and the clinician-selected (operator) settings and protocols. Coordination and synchrony between the patient and ventilator significantly influence patient comfort, treatment effectiveness and homeostasis. Consequently, systems and methods for improving synchrony between patients and ventilators are highly desirable.

Jafari, M., et, al: "Robust Feedback Design for Proportional Assist Ventilation-System Dynamics and Problem Definition": Decision and Control, 2005 and "2005 European Control Conference, CDC-E CC '05, 44th IEEE Conference, Seville, Spain, 12-15 Dec. 2005, Piscataway, NJ, USA, IEEE, 12 December 2005, pages 4839-4844, XP 010884460 ISBN: 987-0-7803-9567-1 (D1) discloses to predict patient effort and to control a ventilator based on an equation of motion, using periodic functions for modelling breath.

WO 2002/028460 discloses to determine a leak flow value by equating the leak flow to a leak factor times a square root of a patient pressure differential. In the present application, the leak flow additionally accounts for an elastic leak source, wherein the elastic leak source cross sectional area is proportional to the patient pressure differential.

SUMMARY

A method and a system according to the pending independent claims are provided.

Systems and methods are described for efficient, continuous and online computation of patient respiratory muscle effort. According to one embodiment, a method is provided for configuring and operating a ventilation system based on an estimated physiologic respiratory muscle effort value or other parameters derived therefrom for monitoring or breath delivery purposes. Patient-ventilator characteristics representing values of parameters of interest associated with static or dynamic properties or attributes of a ventilated patient system are received, estimated and/or measured. The ventilated patient system includes a respiratory subsystem of a patient and a ventilation system, which delivers a flow of gas to the patient. Online quantification of respiratory muscle effort of the patient is continuously performed by (i) establishing a respiratory predictive model of the ventilated patient system based on an equation of motion and functions that approximate clinically-observed, patient-generated muscle pressures, (ii) determining an instantaneous leak flow value for the ventilated patient system, and (iii) based on the patient-ventilator characteristics and the instantaneous leak flow value, solving the respiratory predictive model to extract an estimated physiologic respiratory muscle effort (muscle pressure) value. Then, based on the estimated physiologic respiratory muscle effort value or other parameters derived therefrom the ventilation system is configured and operated for monitoring or breath delivery purposes.

In the aforementioned embodiment, the functions may be periodic or semi-periodic functions having constant or time-varying amplitudes.

In various instances of the aforementioned embodiments, the functions that approximate clinically-observed, patient-generated muscle pressures may include a periodic function for an inspiratory and expiratory phases of respiration that approximates clinically-observed, inspiratory muscle pressures and the estimated physiologic respiratory muscle pressure represents an estimate of inspiratory muscle effort generated by the patient.

In the context of various of the aforementioned embodiments, an exemplary periodic function for the inspiratory phase of respiration may be generally expressed as:

$$P_{musi}(t) = -P_{max}\left(1 - \frac{t}{t_v}\right)\sin\left(\frac{\pi t}{t_v}\right)$$

where,

$P_{max}$ represents a maximum inspiratory muscle pressure, which may be a constant or a time-varying parameter;

$t_v$ represents duration of inspiration; and

t represents an elapsed breath time varying between 0 and a total sum of inspiration and expiration periods.

In various instances of the aforementioned embodiments, the functions that approximate clinically-observed, patient-generated muscle pressures include a periodic function for the expiratory phase of respiration that approximates clinically-observed, expiratory muscle pressures and the estimated physiologic respiratory muscle pressure value represents an estimate of expiratory muscle effort generated by the patient.

In the aforementioned embodiment, an exemplary periodic function for the expiratory phase of respiration may be generally expressed as:

$$P_{mus_e}(t) = P_{max}\left(\frac{t}{t_v}\right)\sin\left(\frac{\pi(t-t_v)}{t_{tot}-t_v}\right)$$

where,

$P_{max}$ represents a maximum expiratory muscle pressure, which may be a constant or a time-varying parameter;

$t_v$ represents duration of expiration;

$t_{tot}$ represents a total sum of inspiration and expiration periods; and t represents an elapsed breath time varying between 0 and $t_{ot}$.

In various instances of the aforementioned embodiments, the respiratory predictive model is assumed to be valid for multiple breath cycles of the patient and the respiratory predictive model is periodically reestablished, updated or optimized at predetermined temporal windows during breath cycles of the patient.

In the context of various of the aforementioned embodiments, solving the respiratory predictive model to extract an estimated physiologic respiratory muscle effort value involves solving the respiratory predictive model during a breath cycle subsequent to establishment of the respiratory predictive model and compensating the estimated physiologic respiratory muscle effort value for time delays introduced by a measurement system and indirect indication of muscular activity by surrogate phenomena.

In the aforementioned embodiment, compensating the estimated physiologic respiratory muscle effort value for time delays involves application of a single-pole dynamic compensation, an example of which may be generally expressed as:

$$P_{mus,\,deliver}(s) = \frac{We^{-s\tau}}{s+z}P_{mus}(s)$$

where,

W represents a scaling factor incorporating a magnitude ratio of actual to delivered muscle pressure;

$\tau$ represents a delay time constant; and

z represents the single pole; and for the inspiration function

$$P_{mus}(s) = (-\pi)\frac{\frac{P_{max}}{t_v}\left(s-\frac{\pi}{t_v}\right)^2}{\left[s^2+\left(\frac{\pi}{t_v}\right)^2\right]^2}\;.$$

In the context of various of the aforementioned embodiments, solving the respiratory predictive model to extract a respiratory muscle effort value includes optimizing derived parameters of the equation of motion on an ongoing basis to tune to dynamics of the ventilated patient system.

In the aforementioned embodiment, the dynamics may include parameters characterizing breathing mechanism and behavior of the patient.

Other embodiments of the present invention provide a ventilator system, which includes a patient-interface through which a flow of gas is delivered to a patient, a patient model estimator and a controller. The patient model estimator is operable to receive measurements or estimates of one or more patient-ventilator characteristics of a ventilated patient system including a respiratory subsystem of the patient and inspiratory and expiratory accessories of the ventilator system. In one embodiment, the patient model estimator performs continuous, online quantification of respiratory muscle effort. In some embodiments, the patient model estimator quantifies patient respiratory muscle effort by (i) establishing a respiratory predictive model of the ventilated patient system based on an equation of motion and one or more periodic or semi-periodic functions that approximate clinically-observed, patient-generated muscle pressures, , and (ii) based on at least the received characteristics, solving the respiratory predictive model to extract a respiratory muscle pressure value. In some embodiments, the quantification of patient respiratory muscle effort further includes determining an instantaneous leak flow value for the ventilated patient system. In other embodiments, solving the respiratory predictive model is further based on the instantaneous leak flow value. The controller is operable to control various aspects of delivery of the flow of gas to the patient based on the ventilator settings and respiratory muscle pressure value and/or one or more other respiratory parameters derived based on the respiratory muscle pressure value.

In some instances of the aforementioned embodiment, the one or more periodic or semi-periodic functions include a periodic or semi-periodic function that approximates clinically-observed, inspiratory muscle pressures and the respiratory muscle pressure value represents an estimate of inspiratory muscle effort generated by the patient.

In various instances of the aforementioned embodiments, an exemplary periodic function for the inspiratory phase of respiration may be generally expressed as:

$$P_{musi}(t) = -P_{\max}\left(1 - \frac{t}{t_v}\right)\sin\left(\frac{\pi t}{t_v}\right)$$

where,
$P_{\max}$ represents a maximum inspiratory muscle pressure;
$t_v$ represents duration of inspiration; and
t represents an elapsed breath time varying between 0 and a total sum of inspiration and expiration periods.

In the context of various of the aforementioned embodiments, the periodic or semi-periodic functions include a periodic or semi-periodic function that approximates clinically-observed, expiratory muscle pressures and the respiratory muscle pressure value represents an estimate of expiratory muscle effort generated by the patient.

In various instances of the aforementioned embodiments, an exemplary periodic function for the expiration is generally expressed as:

$$P_{muse}(t) = P_{\max}\left(\frac{t}{t_v}\right)\sin\left(\frac{\pi(t - t_v)}{t_{tot} - t_v}\right)$$

where,
$P_{\max}$ represents a maximum expiratory muscle pressure;
$t_v$ represents duration of expiration;
$t_{tot}$ represents a total sum of inspiration and expiration periods; and
t represents an elapsed breath time varying between 0 and $t_{tot}$.

In some instances of the aforementioned embodiments, the respiratory predictive model is assumed to be valid for multiple breath cycles of the patient and the respiratory predictive model is periodically reestablished, updated and/or optimized at predetermined temporal windows during breath cycles of the patient.

In the context of various of the aforementioned embodiments, solving the respiratory predictive model to extract a respiratory muscle effort value involves solving the respiratory predictive model during a breath cycle subsequent to establishment of the respiratory predictive model and then correcting the respiratory muscle pressure value to account for time delays introduced by measurement and indirect indication of muscular activity by surrogate phenomena.

In some instances of the aforementioned embodiment, correcting the respiratory muscle pressure value to account for time delays involves application of a single-pole dynamic generally expressed as:

$$P_{mus, deliver}(s) = \frac{We^{-s\tau}}{s + z} P_{mus}(s)$$

where,
W represents a scaling factor incorporating a magnitude ratio of actual to delivered muscle pressure;
$\tau$ represents a delay time constant; and

z represents the single pole; and for the expiration function

$$P_{mus}(s) = (\pi \frac{P_{max}}{t_v(t_{tot} - t_v)}) \frac{t_v\left[s^2 + \left(\frac{\pi}{t_{tot} - t_v}\right)^2\right] + 2s}{\left[s^2 + \left(\frac{\pi}{t_{tot} - t_v}\right)^2\right]^2}.$$

In some circumstances, solving the respiratory predictive model to extract a respiratory muscle effort value involves optimizing derived parameters of the equation of motion.

This summary provides only a general outline of some embodiments of the invention. Many other objects, features, advantages and other embodiments of the invention will become more fully apparent from the following detailed description, the appended claims and the accompanying drawings.

BRIEF DESCRIPTION OF THE DRAWINGS

A further understanding of the various embodiments of the present invention may be realized by reference to the figures which are described in remaining portions of the specification. In the figures, like reference numerals may be used throughout several of the figures to refer to similar components. In some instances, a sub-label consisting of a lower case letter is associated with a reference numeral to denote one of multiple similar components. When reference is made to a reference numeral without specification to an existing sub-label, it is intended to refer to all such multiple similar components.

FIG. 1 depicts a simplified patient-ventilator modular block diagram in accordance with an embodiment of the present invention.

FIG. 2 represents a simplified lumped-parameter analog model for a patient circuit and a single-compartment respiratory system.

FIG. 3 depicts a patient model estimator in accordance with an embodiment of the present invention.

FIG. 4 is a flow diagram illustrating ventilator control processing in accordance with an embodiment of the present invention.

FIG. 5 is a flow diagram illustrating continuous, online quantification of respiratory muscle effort processing in accordance with an embodiment of the present invention.

FIG. 6 is a schematic depiction of a ventilator.

FIG. 7 schematically depicts control systems and methods that may be employed with the ventilator of FIG. 6.

FIGs. 8A and 8B depict exemplary tidal breathing in a patient, and examples of pressure/flow waveforms observed in a ventilator under pressure support with and without leak condition. Under leak condition, the inhalation flow is the total delivered flow including the leak flow and the exhalation flow is the output flow rate measured by the ventilator and excludes the exhaled flow exhausted through the leak.

FIGs. 9A and 9B depict an example embodiment of the patient interface shown in Fig. 6.

FIG. 10 depicts an exemplary method for controlling the ventilator of FIG. 6, including a method for compensating for leaks in ventilator components according to an embodiment.

DETAILED DESCRIPTION OF THE INVENTION

Systems and methods are described for efficient computation of patient respiratory muscle effort. As indicated above, in a ventilated patient system, coordination and synchrony between the patient and ventilator substantially influence patient comfort, treatment effectiveness and homeostasis. Embodiments of the present invention seek to improve synchrony between patients and ventilators by using a computationally efficient model-predictive approach to determining patient respiratory effort using a clinically-based internal model of the patient muscle pressure generator. In some embodiments, the respiratory predictive model includes one or more equations based on a combination of the equation of motion with a model of the inhalation phase or a model of the exhalation phase that are expressed as functions of one or more time parameters. In this manner, after a current respiratory predictive model is established that is valid for a number of breath cycles, subsequent evaluation of the model can be performed in a computationally efficient manner without the need to recalculate the entire model during each sampling interval. In still other embodiments, the computational model accuracy is further increased by compensating for leaks which may occur in the system or ventilation circuit. A variety of leak estimation techniques may be used within the scope of the present invention, including the techniques described in U.S. Provisional Application 61/041,070, entitled "Ventilator Leak Compensation" .

In the following description, for the purposes of explanation, numerous specific details are set forth in order to provide a thorough understanding of embodiments of the present invention. It will be apparent, however, to one skilled in the art that embodiments of the present invention may be practiced without some of these specific details and/or other embodiments may incorporate other details as necessary to realize the design concept and goals in specific platforms with specific characteristics.

Embodiments of the present invention may include various steps, which will be described below. The steps may be performed by hardware components or may be embodied in machine-executable instructions, such as firmware or software, which may be used to cause a general-purpose or special-purpose processor programmed with the instructions to perform the steps. Alternatively, the steps may be performed and/or facilitated by a combination of hardware, software, firmware and/or one or more human operators, such as a clinician.

Embodiments of the present invention may be provided as a computer program product which may include a machine-readable medium having stored thereon instructions which may be used to program a processor associated with a ventilation control system to perform various processing. The machine-readable medium may include, but is not limited to, floppy diskettes, optical disks, compact disc read-only memories (CD-ROMs), and magneto-optical disks, ROMs, random access memories (RAMs), erasable programmable read-only memories (EPROMs), electrically erasable programmable read-only memories (EEPROMs), magnetic or optical cards, flash memory, MultiMedia Cards (MMCs), secure digital (SD) cards, such as miniSD and microSD cards, or other type of media / machine-readable medium suitable for storing electronic instructions. Moreover, embodiments of the present invention may also be downloaded as a computer program product. The computer program may be transferred from a remote computer to a requesting computer by way of data signals embodied in a carrier wave or other propagation medium via a communication link (e.g., a modem or network connection). For example, various subsets of the functionality described herein may be provided within a legacy or upgradable ventilation system as a result of installation of a software option or performance of a firmware upgrade.

While, for convenience, various embodiments of the present invention may be described with reference to a particular ventilation mode, such as PAV, the present invention is also applicable to various other ventilation modes, including, but not limited to Pressure Support, Pressure Control, Volume Control, BiLevel (volume-controlled pressure-regulated) and the like.

As used herein, the terms "connected" or "coupled" and related terms are used in an operational sense and are not necessarily limited to a direct physical connection or coupling. Thus, for example, two devices of functional units may be coupled directly, or via one or more intermediary media or devices. As another example, devices or functional units may be coupled in such a way that information can be passed there between, while not sharing any physical connection one with another. Based on the disclosure provided herein, one of ordinary skill in the art will appreciate a variety of ways in which connection or coupling exists in accordance with the aforementioned definition.

As used herein, the phrases "in one embodiment," "according to one embodiment," and the like generally mean the particular feature, structure, or characteristic following the phrase is included in at least one embodiment of the present invention, and may be included in more than one embodiment of the present invention. Importantly, such phases do not necessarily refer to the same embodiment. If the specification states a component or feature "may", "can", "could", or "might" be included or have a characteristic, that particular component or feature is not required to be included or have the characteristic.

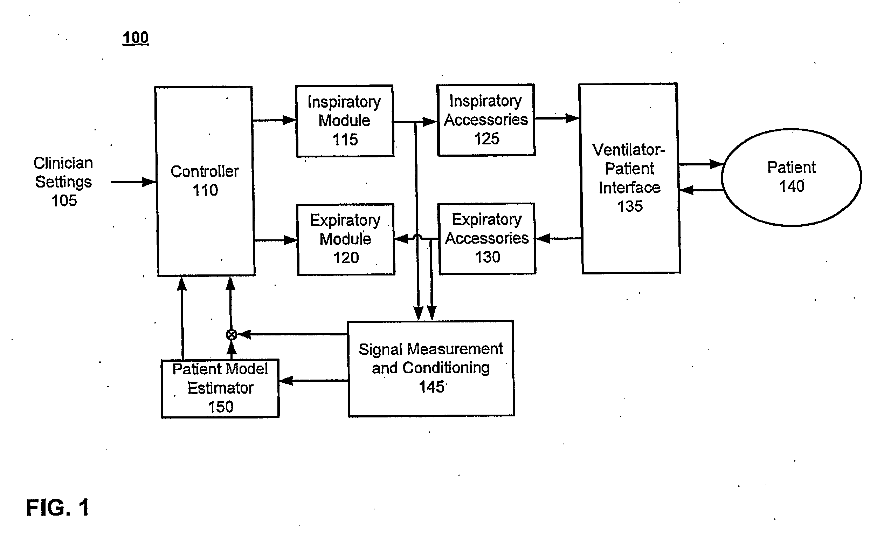

FIG. 1 depicts a simplified patient-ventilator modular block diagram in accordance with an embodiment of the present invention. In the current example, the major functional units / components of a patient-ventilator system 100 are illustrated, including an inspiratory module 115, an expiratory module 120, inspiratory accessories 125, expiratory accessories 130, a ventilator-patient interface 135, a signal measurement and conditioning module 145, a patient model estimator 150, a controller 110 and a patient 140.

The inspiratory module 115 may include a gas source, regulators and various valving components. The expiratory module 120 typically includes an exhalation valve and a heated filter. The inspiratory accessories 125 and the expiratory accessories 130 typically include gas delivery/exhaust circuits and other elements, such as filters, humidifiers and water traps.

Depending upon the particular type of ventilation (e.g., invasive ventilation or noninvasive ventilation), the ventilator-patient interface 135 may include endotracheal tubes or masks or others as appropriate for invasive or non-invasive use as applicable.

Signal measurement and conditioning module 145 receives raw measurement data from various sensors that may be part of the patient-ventilator system, including but not limited to physiological sensors, pressure sensors, flow sensors and the like. The signal measurement and conditioning module 145 may then manipulate various signals in such a way that they meet the requirements of the next stage for further processing. According to one embodiment, the signal measurement and conditioning module 145 may transform the raw sensor measurements into data in a form useable by the patient model estimator 150. For example, pressure and flow sensor data may be digitized and flow sensor data may be integrated to compute delivered volume.

Gas delivered to the patient 140 and/or expiratory gas flow returning from the patient 140 to the ventilation system may be measured by one or more flow sensors (not shown). A flow sensor may comprise any sensor known in the art that is capable of determining the flow of gas passing through or by the sensor. In some particular embodiments of the present invention, the flow sensors may include a proximal flow sensor as is known in the art. In one embodiment, the flow sensors include two separate and independent flow sensors, a first sensor configured to meter a flow of breathing gas delivered to the patient 140 from the ventilation system and a second sensor configured to meter expiratory gas flow returning from the patient 140 to the ventilation system.

According to one embodiment of the present invention, the one or more flow sensors may comprise a single flow sensor positioned at a port defining an entry to an airway of the patient 140. In such an embodiment, the single flow sensor may be configured to meter both a flow of breathing gas delivered to the patient 140 by the ventilation system and a flow of gas returning from the patient 140 to the ventilation system. In one embodiment, a single flow sensor may be located at a connector (e.g., the patient wye) that joins the inspiratory and expiratory limbs of a two-limb patient circuit to the patient airway. Based on the disclosure provided herein, one of ordinary skill in the art will recognize a variety of different types of flow sensors that may be used in relation to different embodiments of the present invention.

During inhalation, the controller 110 commands actuators in the inspiratory module to regulate gas delivery (e.g., flow and oxygen mix) through the ventilator-patient interface 135 responsive to parameter values of a respiratory predictive model continuously evaluated by the patient model estimator 150. For example, in the context of a Proportional Assist Ventilation (PAV) mode, the controller 110 regulates gas delivery such that proximal airway pressure tracks a desired airway trajectory that may be periodically computed based on patient-generated muscle pressure using patient respiratory parameters, instantaneous inspiratory lung flow and clinician settings 105, such as a clinician-set support level. Further description regarding the patient model estimator 150 is provided below.

In one embodiment, the functionality of one or more of the above-referenced functional units may be merged in various combinations. For example, patient model estimator 150 and controller 110 or signal measurement and conditioning module 145 and patient model estimator 150 may be combined. Moreover, the various functional units can be communicatively coupled using any suitable communication method (e.g., message passing, parameter passing, and/or signals through one or more communication paths, etc.). Additionally, the functional units can be physically connected according to any suitable interconnection architecture (e.g., fully connected, hypercube, etc.).

According to embodiments of the invention, the functional units can be any suitable type of logic (e.g., digital logic, software code and the like) for executing the operations described herein. Any of the functional units used in conjunction with embodiments of the invention can include machine-readable media including instructions for performing operations described herein. Machine-readable media include any mechanism that provides (i.e., stores and/or transmits) information in a form readable by a machine (e.g., a computer). For example, a machine-readable medium includes, but is not limited to, read only memory (ROM), random access memory (RAM), magnetic disk storage media, optical storage media or flash memory devices.

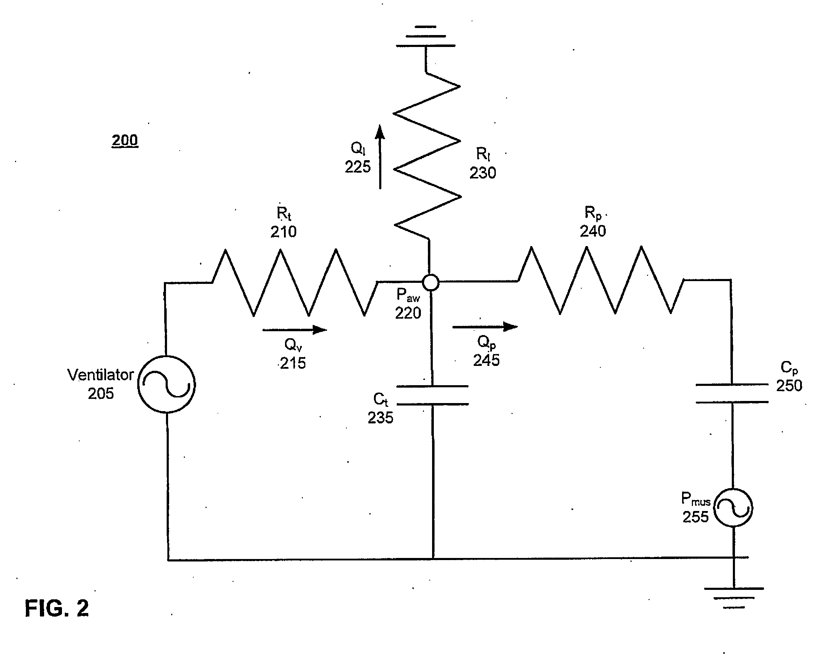

FIG. 2 represents a simplified lumped-parameter analog model for a patient circuit and a single-compartment respiratory system. The model 200 includes a ventilator 205, resistance, $R_t$ 210, representing circuit tubing resistance, compliance, $C_t$ 235, representing circuit tubing compliance, and resistance, $R_1$ 230, representing leak resistance. In the context of this model 200, respiratory dynamics are captured by total respiratory resistance, $R_p$ 240, total respiratory compliance, $C_p$ 250, and patient-generated muscle pressure, $P_{mus}$ 255.

For practical purposes, the magnitude of the negative pressure generated by the inspiratory muscles, $P_{mus}$ 255, is used as an index of breathing effort. Airway pressure, $P_{aw}$ 220, measured at the ventilator-patient interface, e.g., ventilator-patient interface 135, may be calculated on an ongoing basis using patient parameters and $P_{mus}$ 255 according to the equation of motion:

$$P_{aw}(t) = E_p \int Q_p dt + Q_p R_p - P_{mus}(t) \qquad \text{EQ \#1}$$

where,

$$Q_p = Q_{in} - Q_{out} + phase * Q_l \qquad \text{EQ \#2}$$

$Q_p$ 245 is the instantaneous patient flow, and $E_p$ and $R_p$ are the patient's respiratory elastance and resistance, respectively. $Q_{in}$ represents the total flow delivered to the patient wye by the ventilator. $Q_{out}$ is the total flow estimated at the patient wye and exhausted through the exhalation limb. $Q_1$ is the instantaneous leak flow. Phase is - 1 during inspiration and +1 during exhalation. Inspiratory muscle pressure is negative with a magnitude of $P_{mus}$ 255. Patient (lung) flow is assumed positive during inhalation and negative during exhalation.

Constructing an accurate and predictive model of the patient muscle pressure generator is challenging. Inspiratory muscle pressure, $P_{mus}$ 255, is a time-variant excitation function with inter- and intra-subject variations. In normal subjects, it is believed that $P_{mus}$ is in general dependent on breath rate, inspiration time and characteristic metrics of the inspiratory pressure waveform. However, in patients, other factors related to demanded and expendable muscle energy may critically influence muscle pressure generation. For example, for a given peak inspiratory pressure, the maximum sustainable muscle pressure may be affected by factors impairing muscle blood flow (blood pressure, vasomotor tone, muscle tension in the off-phase), the oxygen content of perfusing blood ($P_{o2}$, hemoglobin concentration), blood substrate concentration (glucose, free fatty acids), and the ability to extract sources of energy from the blood. Thus, respiratory motor output may vary significantly in response to variations in metabolic rate, chemical stimuli, temperature, mechanical load, sleep state and behavioral inputs. Moreover, there is a breath-by-breath variability in respiratory output that could lead to tidal volumes varying by a factor of four or more. The mechanism of this variability is not yet known.

According to various embodiments of the present invention, functions that approximate actual clinically-observed inspiratory and expiratory muscle pressures are used as part of a respiratory predictive model by substituting them into the equation of motion (EQ #1) as appropriate. An example of a periodic function meeting these criteria for the inhalation phase is the following:

$$P_{musi}(t) = -P_{max}\left(1 - \frac{t}{t_v}\right)\sin\left(\frac{\pi t}{t_v}\right) \qquad \text{EQ \#3}$$

where,
$P_{max}$ represents a maximum inspiratory pressure,
$t_v$ represents duration of inspiration;
$t$ represents an elapsed breath time varying between 0 and a total sum of inspiration and expiration periods; and
Muscle pressure, $P_{mus}$, represents the magnitude of $P_{mus}$

Based on the disclosure provided herein, one of ordinary skill in the art will recognize a variety of alternative periodic and semi-periodic functions that may be used in relation to different embodiments of the present invention. For example, in EQ #3, above, $P_{max}$ may be assumed to be a constant or a time-varying parameter, thus resulting in a function having a constant amplitude or a time-varying amplitude.

A similar model may be used for the exhalation phase as well. An example of a periodic function meeting the criteria of approximating actual clinically-observed expiratory muscle pressures is the following:

$$P_{muse}(t) = P_{max}\left(\frac{t}{t_v}\right)\sin\left(\frac{\pi(t - t_v)}{t_{tot} - t_v}\right) \qquad \text{EQ \#4}$$

where,

$P_{max}$ represents a maximum expiratory pressure,

$t_v$ represents duration of expiration;

$t_{tot}$ represents a total sum of inspiration and expiration periods;

t represents an elapsed breath time varying between 0 and $t_{tot}$; and

Muscle pressure, $P_{mus}$, represents the magnitude of $P_{mus}$.

Based on the disclosure provided herein, one of ordinary skill in the art will recognize a variety of alternative periodic and semi-periodic functions that may be used in relation to different embodiments of the present invention. For example, in EQ #4, above, $P_{max}$ may be assumed to be a constant or a time-varying parameter, thus resulting in a function having a constant amplitude or a time-varying amplitude.

In alternative embodiments, inspiratory and expiratory resistances used in the respiratory predictive model may be assumed to be equal.

While, as discussed above, under real conditions, $P_{max}$, and $t_v$ are known to demonstrate time-variance, for purposes of various embodiments of the present invention, $P_{max}$ is assumed to be constant for fixed steady state conditions of physiologic and interactive parameters affecting muscle pressure generation. During inspiration, the magnitude of $R_p$ and $C_p$ change dynamically as the lung is inflated.

Taking the Laplace transform of $P_{mus}$ during inspiration to produce a more readily and computationally efficiently solvable algebraic equation yields the following:

$$P_{mus}(s) = (\pi) \frac{\frac{P_{max}}{t_v} (s - \frac{\pi}{t_v})^2}{\left[ s^2 + \left( \frac{\pi}{t_v} \right)^2 \right]^2} \qquad . \qquad \text{EQ \#5}$$

A similar function may be derived for the exhalation phase using EQ #4, above.

In accordance with various embodiments of the present invention, combining the inhalation and exhalation models above with the equation of motion in terms of patient and ventilator/accessories parameters to form a respiratory predictive model, a model-predictive online identification approach is devised to extract $Q_1$ (via a leak detection and characterization algorithm discussed further below), $P_{max}$ and optionally $R_p$ as well as $C_p$.

According to one embodiment, the model-predictive online identification approach involves continuous and breath-by-breath online evaluation and adaptive parameter optimization of the parameters of the equation of motion across the whole breath cycle as well as a number of defined temporal windows during inhalation and active and passive exhalation to constitute a sufficient number of equations to solve for the number of unknowns of interest and/or adequate to optimize one or more derived parameters.

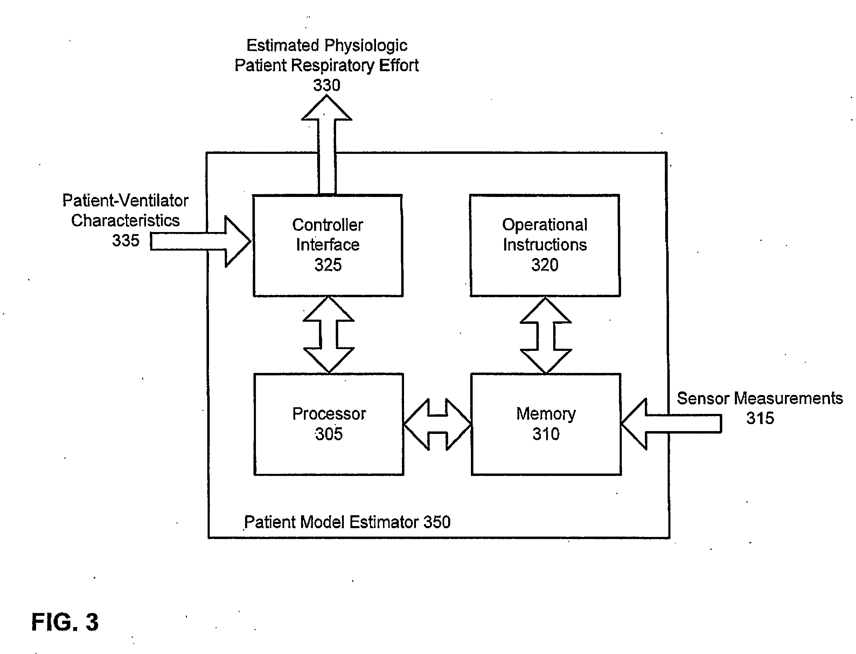

FIG. 3 depicts a patient model estimator 350 in accordance with an embodiment of the present invention that is capable of receiving information and/or parameters regarding various sensor measurements 315, using a computationally efficient model-predictive approach to determining patient respiratory effort using a clinically-based internal model of the patient muscle pressure generator, and providing information regarding estimated physiologic patient respiratory effort 330 to a controller, such as controller 110.

According to the present example, patient model estimator 350 includes a processor 305, a memory 310, operational instructions 320 stored within the memory 310 and a controller interface 325.

Processor 305 may be any processor known in the art that is capable of receiving and processing sensor measurements 315, executing various operational instruction 320 maintained in the memory 310, receiving, measuring and/or estimating patient-ventilator characteristics 335, performing continuous, online quantification of respiratory muscle effort of the patient and otherwise interacting with various other functional units of the ventilator system, such as controller 110 via the controller interface 325. In one embodiment of the present invention, processor 330 may receive interrupts on a periodic basis to trigger ventilator configuration and/or control processing activities. Such interrupts may be received, for example, every 5 milliseconds. Alternatively, the interrupts may be received whenever the validity of various parameter values or the validity of the respiratory predictive model is determined to have expired. Furthermore, interrupts may be received upon availability of sensor measurements 315. Such interrupts may be received using any interrupt scheme known in the art including, but not limited to, using a polling scheme where processor 330 periodically reviews an interrupt register, or using an asynchronous interrupt port of processor 330. Alternatively or additionally, the processor 330 may proactively request sensor measurements 315 be provided from the signal measurement and conditioning module 145 and/or measurements or user input be provided regarding patient-ventilator characteristics 335 on a periodic or as needed basis. Based on the disclosure provided herein, one of ordinary skill in the art will recognize a variety of interrupt and/or polling mechanisms that may be used in relation to different embodiments of the present invention.

In one embodiment of the present invention, processor 330 performs continuous, online quantification of respiratory muscle effort of a patient with reference to a respiratory predictive model of the ventilated patient system as discussed in further detail below. At a high-level, the computationally efficient model-predictive approach to determining patient respiratory effort in accordance with one embodiment of the present invention is generally described as follows. The processor 305 receives operator input indicative of, receives measurements indicative of, or estimates, one or more patient-ventilator characteristics 335. The patient-ventilator characteristics 335 represent values of parameters of interest associated with static or dynamic properties or attributes of the ventilated patient system.

Based on the patient-ventilator characteristics 335 and sensor measurements 315, the processor 305 continuously performs online (i.e., during ventilator operation), quantification of respiratory muscle effort of the patient. Initially, the processor 305 establishes a respiratory predictive model of the ventilated patient system based on the equation of motion and one or more functions that approximate clinically-observed, patient-generated muscle pressures. The respiratory predictive model may be reestablished, updated and/or optimized as described further below.

At each of a predetermined set of computational stages, system leak is characterized and quantified such that a reliable instantaneous leak flow value for the ventilated patient system may be computed. Then, calculations are performed to estimate and/or optimize the rest of the parameters, including one or more of $P_{max}$, $R_p$ and $C_p$. According to one embodiment, the respiratory predictive model is assumed to be valid for multiple breath cycles thereby allowing a model established, updated and/or optimized during one breath cycle to be solved during the same breath cycle or a subsequent breath cycle to extract one or more patient parameters by simply substituting into the current respiratory predictive model (i) received, estimated and/or measured patient-ventilator characteristics 335, (ii) available sensor measurements 315, and (iii) one or more time values, such as the duration of inspiration or expiration, an elapsed breath time and a total sum of inspiration and expiration periods.

In various embodiments, an estimated physiologic respiratory muscle effort value extracted from the model may be compensated for time delays introduced by the ventilator's measurement system and/or the indirect indication of muscular activity by surrogate phenomena (e.g., pressure) by applying a single-pole dynamic described further below.

Finally, information regarding the estimated physiologic patient effort 330 may be provided to the controller 110 via the controller interface 325, thereby configuring and operating the ventilation system based on the estimated physiologic patient effort 330 or other parameters derived there from for monitoring or breath delivery purposes.

Memory 310 includes operational instructions 320 that may be software instructions, firmware instructions or some combination thereof. Operational instructions 320 are executable by processor 305, and may be used to cause processor 305 to deliver information, such as estimated physiologic patient respiratory effort 330 via controller interface 325 to controller 110, which responsive thereto may then control, configure and/or operate the ventilator in a programmed manner based directly or indirectly upon the estimated physiologic patient respiratory effort 330.

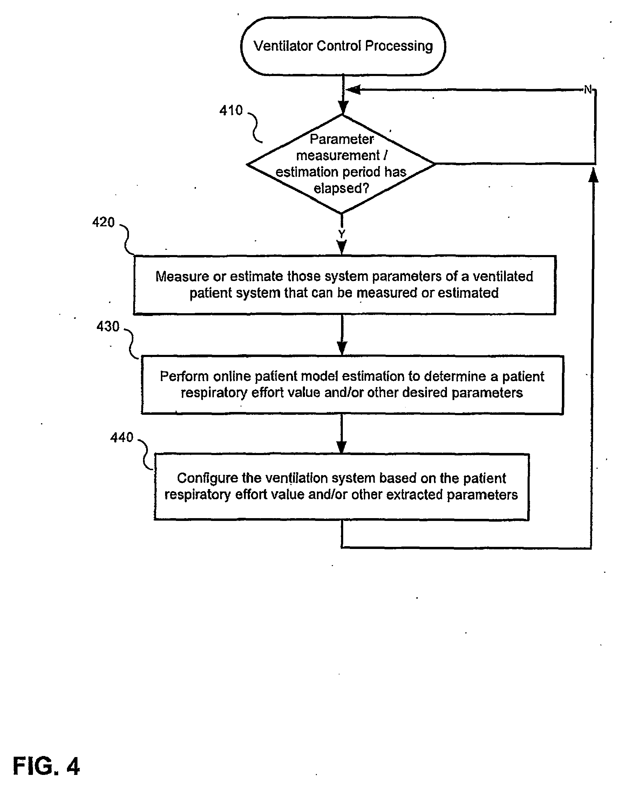

FIG. 4 is a flow diagram illustrating ventilator control processing in accordance with an embodiment of the present invention. According to the present example, an interrupt mechanism and/or polling loop that may be used in accordance with an embodiment of the present invention to initiate patient model estimation and ventilator control processing. In the present example, it is assumed that the interrupt or polling cycle occurs more frequently than a predetermined or configurable parameter measurement/estimation period.

At decision block 410, a determination is made regarding whether the parameter measurement/estimation period has elapsed. If so, then processing continues with block 420; otherwise, processing branches back to decision block 410.

At block 420, depending upon the sensors and data available in the ventilated patient system, measurements and/or estimates of those system parameters capable of being measured or estimated and which are of relevance to patient model estimation are performed. For example, if flow sensors are available in the ventilated patient system, then $Q_{in}$ and/or $Q_{out}$ may be provided to the patient model estimation process. Alternatively or additionally, operator provided inputs regarding one or more system parameters may be collected for purposes of facilitating the patient model estimation process.

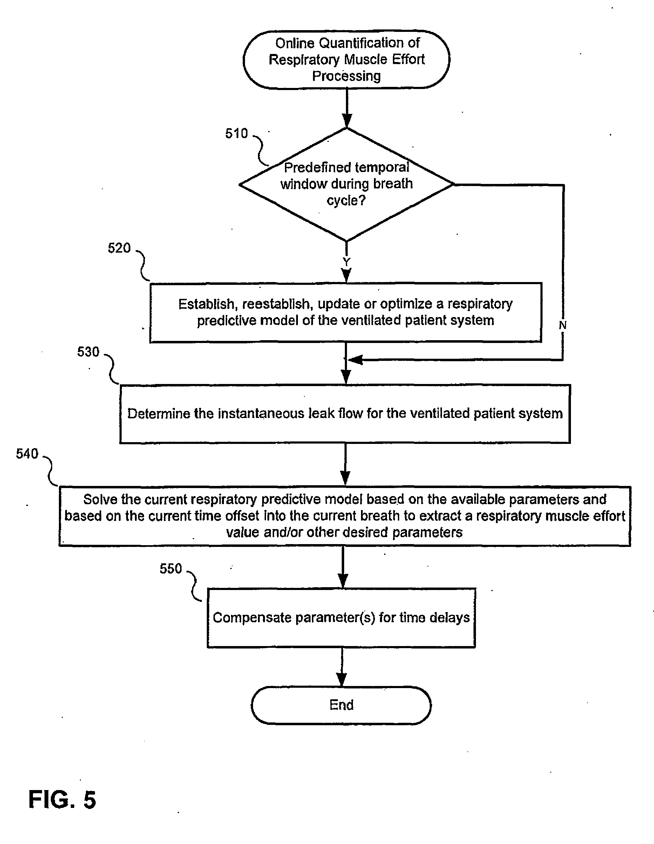

At block 430, an online patient model estimation process is performed to determine an estimated physiologic patient respiratory effort value and potentially other parameters, such as $R_p$ and $C_p$. As will be described further below with reference to FIG. 5, in one embodiment, the patient model estimation process may involve establishment, reestablishment, updating and/or optimization of a respiratory predictive model valid for multiple breath cycles based upon a combination of the equation of motion with functions that substantially approximate clinically-observed, patient-generated muscle pressures. Further details regarding the patient model estimation process are provided below. At this point in the discussion, it is sufficient to simply note that outputs of the patient model estimation process include one or more parameters, e.g., $Q_1$, $P_{max}$, $R_p$ and $C_p$, extracted from the current respiratory predictive model that may be used to directly or indirectly configure operation of the ventilation system.

At block 440, the ventilation system is configured based on the estimated physiologic patient respiratory effort value, other parameters derived or estimated based on the patient model estimation process and/or other respiratory parameters derived based on the estimated physiologic patient respiratory effort value. According to one embodiment, configuration of the ventilation system is accomplished indirectly by the patient model estimator 150 providing one or more outputs of its processing to the controller 110. Controller 110 may then use the one or more parameters provided by patient model estimator 150 to start or stop or regulate a ventilator assisted / supported breath phase or ventilatory parameter, such as to determine an appropriate pressure for a PAV mode, for example.

FIG. 5 is a flow diagram illustrating online quantification of respiratory muscle effort processing that may be performed in a continuous manner in accordance with an embodiment of the present invention. According to the current example, a patient model estimation process is periodically performed responsive to an interrupt mechanism and/or polling loop.

At decision block 510, it is determined whether the current time offset into the breath cycle corresponds to a predefined temporal window during the breath cycle. If so, then processing continues with block 520; otherwise, processing branches to block 530. Examples of predefined temporal windows include, but are not limited to, (i) times during a breath cycle in which characteristics of the breath waveform are known; (ii) times at which sufficiently definite information is available regarding one or more patient or system parameters, (iii) predefined or configurable intervals within a breath cycle (e.g., X times per breath cycle), (iv) times at which sufficiently definite information is available regarding one or more patient parameters or characteristics of breathing behavior based on physiologic knowledge of respiration mechanism and/or expected or reasonable deductions derived from operator inputs and settings and the like. Alternatively, the respiratory predictive model may be reestablished, updated and/or optimized responsive to observing or being informed of changes in patient behavior or patient lung characteristics. The respiratory predictive model may also be reestablished or updated responsive to an error threshold being exceeded or observing or being informed of the fact that one or more patient and/or system parameters derived based on the current respiratory predictive model fall outside of an expected range or otherwise exhibit indicators of inaccuracy.

At block 520, a respiratory predictive model of the ventilated patient system is established, reestablished, updated and/or optimized. According to one embodiment, the respiratory predictive model is one or more equations based on a combination of the equation of motion with a model of the inhalation phase or a model of the exhalation phase that are expressed as functions of one or more time parameters (e.g., $t$, $t_v$ and/or $t_{tot}$). Advantageously, in this manner, after a current respiratory predictive model is established that is valid for a number of breath cycles, subsequent evaluation of the model can be performed in a computationally efficient manner without the need to recalculate the entire model during each sampling interval.

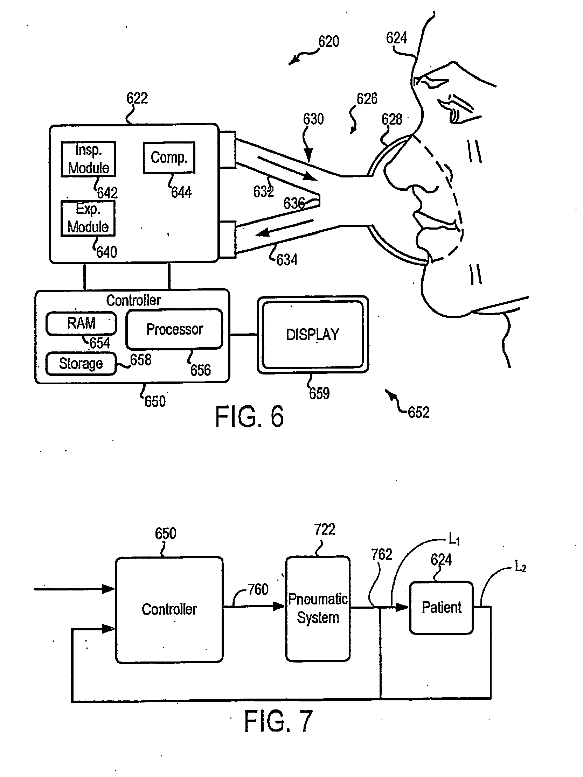

At block 530, the instantaneous leak flow, $Q_1$, for the ventilated patient system is determined. Various methods may be used. According to one embodiment the instantaneous leak flow is determined as described further below with reference to FIGs. 6-10.

At block 540, the current respiratory predictive model is solved based on the available / known parameters and based on the current time offset into the current breath to extract an estimated physiologic respiratory muscle pressure value and/or other desired parameters, such as $R_p$ and $C_p$.

Depending upon the particular ventilator platform, various other approaches to solving the equation of motion in the context of the respiratory predictive model described herein may be used. For example, $R_p$ and $C_p$ may first be calculated and then $P_{max}$ extracted. Alternatively, the respiratory predictive model may be solved during multiple successive sampling intervals or specified temporal windows and the error can be minimized to find the best values. In other approaches, the respiratory predictive model may be solved during particular windows of time during a breath cycle in which characteristics of the breath waveform are known and can therefore be used to verify the extracted parameters.

There are multitude of approaches for identification and estimation of the parameters of the patient-ventilator model (e.g., $R_p$, $C_p$, $P_{max}$, etc.). Selection of an approach is dependent on the characteristics of the operating platform (ventilator system) and performance requirements as well as computational costs. In general, the physical equations governing the dynamical functioning and performance of the system (for example, equation of motion) as well as conservation laws such as mass and volume balance over cyclical respiratory intervals (e.g., one complete breath period) may be used to determine the unknown parameters of interest. In addition, the closed-loop nature of ventilatory functions, namely, feedback control and maintenance of pre-set pressure and/or flow trajectories with known expected characteristics (e.g., constant slope), may be used to generate additional equations and mathematical relationships. Furthermore, such equations and mathematical relationships may be applied under appropriately conditioned temporal windows in conjunction with expected dynamics of the respiration function to solve for or retune or optimize parameters on interest.

In one embodiment, estimates of $R_p$, $C_p$ may be available (provided by the operator) or derived during ventilation using protocols and algorithms for respiratory maneuvers and procedures (e.g., controlled test breaths) to determine and tune respiratory mechanics ($R_p$, $C_p$, etc.). The estimated values for $R_p$, $C_p$ may then be used in the equation of motion and applied at one or several points during inhalation and exhalation to determine an optimum estimate of the corresponding $P_{max}$.

In other embodiments, after a feasible approach for the platform and application of interest is selected, a set of equations may be determined to be applied using a cost effective methodology for online parameter estimation and optimization (e.g., methods and algorithms for closed-loop identification, neural networks and neurodynamic programming, adaptive parameter estimation, etc.). Following an appropriate online estimation of choice selected specifically to satisfy the design needs of specific projects, one or more model parameters ($R_p$, $C_p$, $P_{max}$) may be estimated and regularly updated as need be.

FIG. 6 depicts a ventilator 620 according to the present description. As will be described in detail, the various ventilator system and method embodiments described herein may be provided with control schemes that provide improved leak estimation and/or compensation. These control schemes typically model leaks based upon factors that are not accounted for in prior ventilators, such as elastic properties and/or size variations of leak-susceptible components. The present discussion will focus on specific example embodiments, though it should be appreciated that the present systems and methods are applicable to a wide variety of ventilator devices.

Referring now specifically to FIG. 6, ventilator 620 includes a pneumatic system 622 for circulating breathing gases to and from patient 624 via airway 626, which couples the patient to the pneumatic system via physical patient interface 628 and breathing circuit 630. Breathing circuit 630 could be a two-limb or one-limb circuit for carrying gas to and from the patient. A wye fitting 636 may be provided as shown to couple the patient interface to the breathing circuit.

The present systems and methods have proved particularly advantageous in non-invasive settings, such as with facial breathing masks, as those settings typically are more susceptible to leaks. However, leaks do occur in a variety of settings, and the present description contemplates that the patient interface may be invasive or non-invasive, and of any configuration suitable for communicating a flow of breathing gas from the patient circuit to an airway of the patient. Examples of suitable patient interface devices include a nasal mask, nasal/oral mask (which is shown in FIG. 6), nasal prong, full-face mask, tracheal tube, endotracheal tube, nasal pillow, etc.

Pneumatic system 622 may be configured in a variety of ways. In the present example, system 622 includes an expiratory module 640 coupled with an expiratory limb 634 and an inspiratory module 642 coupled with an inspiratory limb 632. Compressor 644 is coupled with inspiratory module 642 to provide a gas source for ventilatory support via inspiratory limb 632.

The pneumatic system may include a variety of other components, including sources for pressurized air and/or oxygen, mixing modules, valves, sensors, tubing, accumulators, filters, etc. Controller 650 is operatively coupled with pneumatic system 622, signal measurement and acquisition systems, and an operator interface 652 may be provided to enable an operator to interact with the ventilator (e.g., change ventilator settings, select operational modes, view monitored parameters, etc.). Controller 650 may include memory 654, one or more processors 656, storage 658, and/or other components of the type commonly found in command and control computing devices. As described in more detail below, controller 650 issues commands to pneumatic system 622 in order to control the breathing assistance provided to the patient by the ventilator. The specific commands may be based on inputs received from patient 624, pneumatic system 622 and sensors, operator interface 652 and/or other components of the ventilator. In the depicted example, operator interface includes a display 659 that is touch-sensitive, enabling the display to serve both as an input and output device.

FIG. 7 schematically depicts exemplary systems and methods of ventilator control. As shown, controller 650 issues control commands 760 to drive pneumatic system 722 and thereby circulate breathing gas to and from patient 624. The depicted schematic interaction between pneumatic system 722 and patient 624 may be viewed in terms of pressure and/or flow "signals." For example, signal 762 may be an increased pressure which is applied to the patient via inspiratory limb 632. Control commands 760 are based upon inputs received at controller 650 which may include, among other things, inputs from operator interface 652, and feedback from pneumatic system 722 (e.g., from pressure/flow sensors) and/or sensed from patient 624.

In many cases, it may be desirable to establish a baseline pressure and/or flow trajectory for a given respiratory therapy session. The volume of breathing gas delivered to the patient's lung and the volume of the gas exhaled by the patient are measured or determined, and the measured or predicted/estimated leaks are accounted for to ensure accurate delivery and data reporting and monitoring. Accordingly, the more accurate the leak estimation, the better the baseline calculation of delivered and exhaled volume as well as event detection (triggering and cycling phase transitions).

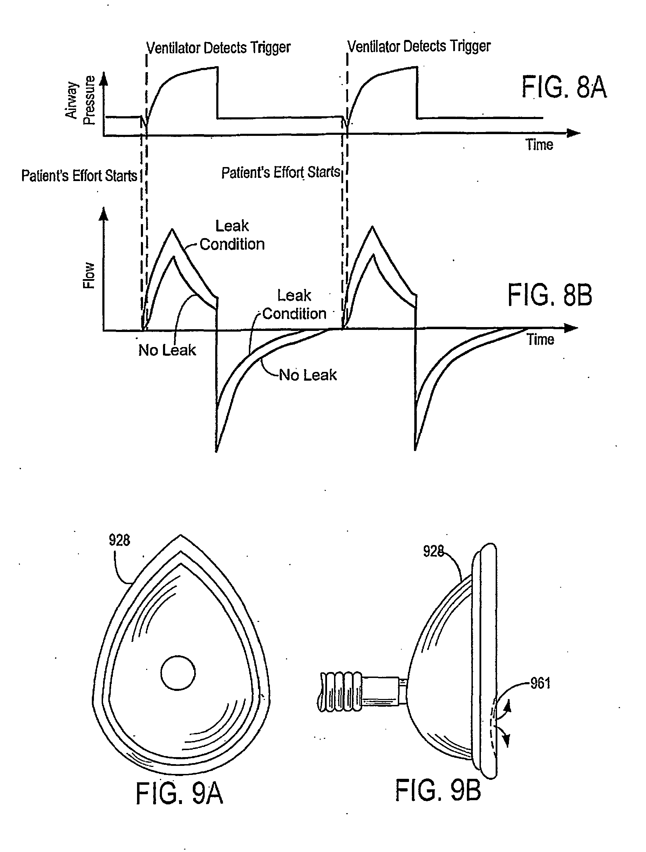

FIGs. 7, 8A and 8B may be used to illustrate and understand leak effects and errors. As discussed above, therapy goals may include generating a desired time-controlled pressure within the lungs of patient 624, and in patient-triggered and -cycled modes, achieve a high level of patient-device synchrony.

FIG. 8A shows several cycles of flow/pressure waveforms spontaneous breathing under Pressure Support mode with and without leak condition. As discussed above, a patient may have difficulty achieving normal tidal breathing, due to illness or other factors.

Regardless of the particular cause or nature of the underlying condition, ventilator 620 typically provides breathing assistance during inspiration and exhalation. FIG. 8B shows an example of flow waveform under Pressure Support in presence of no leak as well as leak conditions. During inspiration more flow is required (depending on the leak size and circuit pressure) to achieve the same pressure level compared to no leak condition. During exhalation, a portion of the volume exhaled by the patient would exit through the leak and be missed by the ventilator exhalation flow measurement subsystem. In many cases, the goal of the control system is to deliver a controlled pressure or flow profile or trajectory (e.g., pressure or flow as a function of time) during the inspiratory phases of the breathing cycle. In other words, control is performed to achieve a desired time-varying pressure or flow output 762 from pneumatic system 722, with an eye toward causing or aiding the desired tidal breathing shown in FIG. 8A.

Improper leak accounting can compromise the timing and magnitude of the control signals applied from controller 650 to pneumatic system 722 especially during volume delivery. Also, lack or inaccurate leak compensation can jeopardize spirometry and patient data monitoring and reporting calculations. As shown at schematic leak source $L_1$, the pressure applied from the pneumatic system 722 to patient interface 628 may cause leakage of breathing gas to atmosphere. This leakage to atmosphere may occur, for example, at some point on inspiratory limb 632 or expiratory limb 634, or at where breathing circuit 630 couples to patient interface 628 or pneumatic system 722.

In the case of non-invasive ventilation, it is typical for some amount of breathing gas to escape via the opening defined between the patient interface (e.g., facial breathing mask) and the surface of the patient's face. In facial masks, this opening can occur at a variety of locations around the edge of the mask, and the size and deformability of the mask can create significant leak variations. As one example, as shown in FIG. 9A and FIG. 9B, the facial breathing mask may be formed of a deformable plastic material with elastic characteristics. Under varying pressures, during inspiration and expiration the mask may deform, altering the size of the leak orifice 961. Furthermore, the patient may shift (e.g., talk or otherwise move facial muscles), altering the size of leak orifice 961. Due to the elastic nature of the mask and the movement of the patient, a leak compensation strategy assuming a constant size leak orifice may be inadequate.

Accurately accounting for the magnitude of leak $L_1$ may provide significant advantages. In order for controller 650 to command pneumatic system 722 to deliver the desired amount of volume/pressure to the patient at the desired time and measure/estimate the accurate amount of gas volume exhaled by the patient, the controller must have knowledge of how large leak $L_1$ is during operation of the ventilator. The fact that the leak magnitude changes dynamically during operation of the ventilator introduces additional complexity to the problem of leak modeling.

Triggering and cycling (patient-ventilator) synchrony may also be compromised by sub-optimal leak estimation. In devices with patient-triggered and patient-cycled modalities that support spontaneous breathing efforts by the patient, it can be important to accurately detect when the patient wishes to inhale and exhale. Detection commonly occurs by using accurate pressure and/or lung flow (flow rates into or out of the patient lung) variations. Leak source $L_2$ represents a leak in the airway that causes an error in the signals to the sensors of pneumatic system 722. This error may impede the ability of ventilator to detect the start of an inspiratory effort, which in turn compromises the ability of controller 650 to drive the pneumatic system in a fashion that is synchronous with the patient's spontaneous breathing cycles.

In some embodiments, leak estimation is included when quantifying the patient respiratory muscle effort and/or when controlling the delivery of gas to the patient. While a variety of leak estimation and leak calculation techniques may be used within the scope of the present invention, in some embodiments leak calculation is performed in a manner similar to that described in U.S. Provisional Application 61/041,070 . Improved leak estimation may be achieved in the present examples through provision of a control scheme that more fully accounts for factors affecting the time-varying magnitude of leaks under interface and airway pressure variations. The present example may include, in part, a constant-size leak model consisting of a single parameter (orifice resistance, leak conductance, or leak factor) utilized in conjunction with the pneumatic flow equation through a rigid orifice, namely,

$$Q_{leak} = (\text{leak factor/Resistance/Conductance}) * \sqrt{\Delta P} \qquad \text{EQ \#6}$$

Where $\Delta P$ = pressure differential across the leak site. This assumes a fixed size leak (i.e., a constant leak resistance or conductance or factor over at least one breath period).

To provide a more accurate estimate of instantaneous leak, the leak detection system and method may also take into account the elastic properties of one or more components of the ventilator device (e.g., the face mask, tubing used in the breathing circuit, etc.). This more accurate leak accounting enhances patient-ventilator synchrony and effectiveness under time-varying airway pressure conditions in the presence of both rigid orifice constant size leaks as well as pressure-dependent varying-size elastic leak sources.

According to the pneumatic equations governing the flow across an orifice, the flow rate is a function of the area and square root of the pressure difference across the orifice as well as gas properties. For derivation of the algorithm carried out by the controller, constant gas properties are assumed and a combination of leak sources comprising of rigid fixed-size orifices (total area = $A_r$ = constant) and elastic opening through the patient interface [total area = $A_e$ ($P$) = function of applied pressure].
Therefore,

$$Q_{leak} = K_o * (A_r + A_e(P)) * \sqrt{\Delta P} \qquad\qquad EQ\ \#7$$

$K_o$ = assumed constant

For the purposes of this implementation, at low pressure differences, the maximum center deflection for elastic membranes and thin plates are a quasi-linear function of applied pressure as well as dependent on other factors such as radius, thickness, stress, Young's Modulus of Elasticity, Poisson's Ratio, etc. Therefore,

$$A_e(P) = K_e * \Delta P \qquad\qquad EQ\ \#8$$

$K_e$ = assumed constant

As $\Delta P$ is the pressure difference across a leak source to ambient ($P_{ambient}$ = 0), then we substitute $\Delta P$ by the instantaneous applied pressure $P(t)$ and rearrange EQ #6 as follows ($K_1$ and $K_2$ are assumed to be constant):

$$Q_{leak} = K_0(A_r + K_e P(t))\sqrt{\Delta P} \qquad\qquad EQ\ \#9$$

$$Q_{leak} = K_1 * P(t)^{1/2} + K_2 * P(t)^{3/2} \qquad\qquad EQ\ \#10$$

Also, the total volume loss over one breath period = $V_{leak}$ = Delivered Volume -Exhausted Volume;

$$V_{leak} = \int_0^{Tb}[K_1 P(t)^{1/2} + K_2 P(t)^{3/2}]dt = \int_0^{Tb}[Q_{delivered} - Q_{exh}] * dt \qquad\qquad EQ\ \#11$$

$T_b$= full breath period

The general equation of motion for a patient ventilator system during passive exhalation can then be written,

$$P_{aw} + P_m = R * (Q_{leak} + Q_{exh} - Q_{delivered}) + (1/C) * \int[Q_{leak} + Q_{exh} - Q_{delivered}] * dt$$

$$EQ\ \#12$$

$P_{aw}$ = airway pressure
$P_m$ = muscle pressure
$R$ = resistance
$C$ = Compliance

Assuming that when end exhalation conditions are present a constant airway pressure is being delivered (steady PEEP), constant bias flow maintained during exhalation phase $Q_{delivered}$, constant leak flow (due to no pressure variation), and $P_m$ = 0 (due to no patient respiratory effort), the equation of motion could be differentiated and reorganized as follows:

$$\frac{dP_{aw}}{dt} = 0 = R * Q_{exh}dot + \frac{Q_{leak} + Q_{exh} - Q_{delivered}}{C}$$

EQ #13

$$Q_{leak} = (Q_{delivered} - Q_{exh}) - R * C * Q_{exh}dot$$

EQ #14

$Q_{exh}dot$ = time derivative of exhausted flow

If $Q_{exh}dot = 0$, EQ #13 can be reduced to

$$Q_{leak} = Q_{delivered} - Q_{exh}$$

EQ #15

And subsequently,

$$Q_{leak} = K_1(PEEP)^{1/2} + K_2(PEEP)^{3/2}$$

EQ #16

Otherwise $Q_{exh}$ dot $\neq$ 0. In this case, an appropriate duration of time $\Delta T$ is taken during passive exhalation period and assuming constant delivered flow, equation can be derived as follows:

$$R * C = \frac{(Q_{exh}(t + \Delta T) - Q_{exh}(t)}{(Q_{exh}dot(t + \Delta T) - Q_{exh}dot(t)}$$

EQ #17

And,

$$Q_{leak}(t_i + \Delta T) = K_1(PEEP)^{1/2} + K_2(PEEP)^{3/2} =$$
$$[Q_{delivered}(t_i + \Delta T) - Q_{exh}(ti + \Delta T)] - R * C * Q_{exh}dot(ti + \Delta T)$$

EQ #18

Therefore, EQ #11 and EQ #15 and EQ #18 may be used to solve for $K_1$ and $K_2$. These calculations may be repeated every breath cycle and applied over appropriate time windows (i.e. during exhalation) and breathing conditions to optimize parameter estimation and minimize the total error between estimated total volume loss and actual measured volume loss across the full breath cycle. The constants $K_1$ and $K_2$ may be stored and compared to track changes and update various parameters of the system such as the triggering and cycling sensitivities, etc.

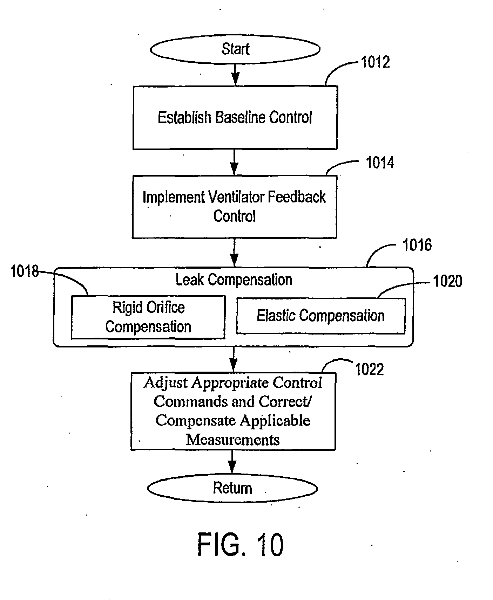

FIG. 10 shows an exemplary control strategy that may be implemented by the controller 650 to increase the accuracy and timing of the baseline breathing assistance provided by ventilator 620 and pneumatic system 722 for a variety of respiratory therapies. In this example, the method is repeated periodically every breathing cycle. In other examples, the dynamic updating of leak estimation may occur more or less than once per patient breathing cycle.

At block 1012, the routine establishes a baseline level of leak estimation and compensation. This may be a preset value stored in the controller 650 or may be updated taking into account various parameters of the breathing cycle and ventilator 620, such as the Positive End Expiratory Pressure PEEP, the set inspiratory pressure or flow/volume targets, the volumetric airflow delivered by pneumatic system 722, and type of the breathing circuit 630, etc.

The routine then proceeds to block 1014 where the feedback control (e.g., as shown in FIG. 8) is implemented. Various control regimes may be implemented, including pressure, volume and/or flow regulation. Control may also be predicated on inputs received from the patient, such as pressure variations in the breathing circuit which indicate commencement of inspiration. Inputs applied via operator interface 652 may also be used to vary the particular control regime used. For example, the ventilator may be configured to run in various different operator-selectable modes, each employing different control methodologies.

The routine advances to block 1016 where the leak compensation is performed. Various types of leak compensation may be implemented. For example, as shown at block 1018, rigid-orifice compensation may be employed using values calculated as discussed above. In particular, holes or other leak sources may be present in non-elastic parts of the breathing circuit, such as the ports of a facial mask (not shown) and/or in the inspiratory and expiratory limbs. EQ #6 may be used to calculate the volumetric airflow through such an orifice, assuming the leak factor/resistance/conductance is constant.

Elastic properties of ventilator components may also be accounted for during leak compensation, as shown at block 1020, for example using values calculated as described above. Specifically, elastic properties of patient interface 628 and/or breathing circuit 630 may be established (e.g., derived based on material properties such as elastic modulus, Poisson's ratio, etc.), and employed in connection with calculations such as those discussed above in reference to EQ #11, 15 and/or 18, to account for the deformation of orifice 961, as shown in FIG. 9B. Using these example calculations, constants $K_1$ and $K_2$ may be solved for and updated dynamically to improve the accuracy of leak estimation. In alternate implementations, the method may use any suitable alternate mechanism or models for taking into account the elastic properties of a ventilator component having a leak-susceptible orifice.

The routine then proceeds to block 1022 where appropriate baseline control commands and measurements are adjusted to compensate for the leaks in the ventilator calculated in 1016 i.e., adjust appropriate control command and correct and/or compensate applicable measurements. In many settings, it will be desirable to regularly and dynamically update the compensation level (e.g., once every breathing cycle) in order to optimize the control and compensation.

In conclusion, embodiments of the present invention provide novel systems, methods and devices for improving synchrony between patients and ventilators by employing a computationally efficient model-predictive approach to determining patient respiratory effort using a clinically-based internal model of the patient muscle pressure generator. While detailed descriptions of one or more embodiments of the invention have been given above, various alternatives, modifications, and equivalents will be apparent to those skilled in the art.

Therefore, the above description should not be taken as limiting the scope of the invention, which is defined by the appended claims.

**Claims**

**1.** A method comprising:

receiving, measuring, or estimating one or more patient-ventilator characteristics representing values of parameters of interest associated with static or dynamic properties or attributes of a ventilated patient system (100), the ventilated patient system (100) including a respiratory subsystem of a patient (140, 624) and a ventilation system (620), which delivers a flow of gas to the patient;
performing quantification of respiratory muscle effort of the patient by

i) establishing a respiratory predictive model of the ventilated patient system based on an equation of motion and one or more functions that approximate clinically-observed, patient-generated muscle pressures,

**characterized in that**:

performing quantification of the respiratory muscle effort of the patient further comprises:

ii) determining an instantaneous leak flow value for the ventilated patient system, including a leak flow value for constant size leak opening and a pressure-dependent varying-size leak opening, and
iii) based on the one or more patient-ventilator characteristics and the instantaneous leak flow value, solving the respiratory predictive model to extract an estimated physiologic respiratory muscle effort value; and

the method further comprises the step of configuring and operating the ventilation system based on the estimated physiologic respiratory muscle effort value or other parameters derived therefrom for monitoring or breath delivery purposes.

**2.** The method of claim 1, wherein the one or more functions comprise periodic or semi-periodic functions.

**3.** The method of claim 2, wherein the periodic or semi-periodic functions have constant amplitudes,
and/or
wherein the one or more periodic or semi-periodic functions have time-varying amplitudes.

**4.** The method of claim 1, wherein the one or more functions that approximate clinically-observed, patient-generated muscle pressures include a periodic inspiratory function for an inspiratory phase of respiration that approximates clinically-observed, inspiratory muscle pressures and the estimated physiologic respiratory muscle effort value comprises an estimate of inspiratory muscle effort generated by the patient.

**5.** The method of claim 4, wherein the periodic inspiratory function is expressed as:

$$P_{musi}(t) = -P_{\max}\left(1 - \frac{t}{t_v}\right)\sin\left(\frac{\pi t}{t_v}\right)$$

where,
$P_{\max}$ represents a maximum inspiratory muscle pressure, which may be a constant or a time-varying parameter;
$t_v$ represents duration of inspiration; and
t represents an elapsed breath time varying between 0 and a total sum of inspiration and expiration periods.

**6.** The method of claim 1, wherein the one or more functions that approximate clinically-observed, patient-generated muscle pressures include a periodic expiratory function for an expiratory phase of respiration that approximates clinically-observed, expiratory muscle pressures and the estimated physiologic respiratory muscle effort value comprises an estimate of expiratory muscle effort generated by the patient.

**7.** The method of claim 6, wherein the periodic expiratory function is expressed as:

$$P_{muse}(t) = P_{\max}\left(\frac{t}{t_v}\right)\sin\left(\frac{\pi(t - t_v)}{t_{tot} - t_v}\right)$$

where,
$P_{\max}$ represents a maximum expiratory muscle pressure, which may be a constant or a time-varying parameter;
$t_v$ represents duration of expiration;
$t_{tot}$ represents a total sum of inspiration and expiration periods; and
t represents an elapsed breath time varying between 0 and $t_{tot}$.

**8.** The method of claim 5, wherein the respiratory predictive model is assumed to be valid for a plurality of breath cycles of the patient and the method further comprises periodically reestablishing, updating or optimizing the respiratory predictive model at predetermined temporal windows during breath cycles of the patient.

**9.** The method of claim 8, wherein said solving the respiratory predictive model to extract an estimated physiologic respiratory muscle effort value comprises solving the respiratory predictive model during a breath cycle of the plurality of breath cycles subsequent to establishment of the respiratory predictive model and compensating the estimated physiologic respiratory muscle effort value for time delays introduced by a measurement system and indirect indication of muscular activity by surrogate phenomena.

**10.** The method of claim 9 and claim 7, wherein said compensating the estimated physiologic respiratory muscle effort value for time delays involves application of a single-pole dynamic expressed as:

$$P_{mus, deliver(s)} = \frac{We^{-s\tau}}{s + z} P_{mus(s)}.$$

where,

W represents a scaling factor incorporating a magnitude ratio of actual to delivered muscle pressure;

$\tau$ represents a delay time constant; and

z represents the single pole; and

$$P_{mus}(s) = (\pi) \frac{\dfrac{P_{max}}{t_v}(s - \dfrac{\pi}{t_v})^2}{\left[ s^2 + \left( \dfrac{\pi}{t_v} \right)^2 \right]^2} \; ;$$

for inspiration

and,

$$Pmus(s) = (\pi \frac{P_{max}}{t_v(t_{tot} - t_v)}) \frac{t_v \left[ s^2 + \left( \dfrac{\pi}{t_{tot} - t_v} \right)^2 \right] + 2s}{\left[ s^2 + \left( \dfrac{\pi}{t_{tot} - t_v} \right)^2 \right]^2}$$

for exhalation.

11. The method of claim 1, wherein said solving the respiratory predictive model to extract a respiratory muscle effort value includes optimizing derived parameters of the equation of motion on an ongoing basis to tune to dynamics of the ventilated patient system.

12. The method of claim 11, wherein the dynamics include breathing behavior of the patient.

13. A ventilator system (100) comprising:

a ventilator-patient interface (628) through which a flow of gas is delivered to a patient (140, 624);
a patient model estimator (150, 350) operable to receive measurements or estimates of one or more patient-ventilator characteristics of the ventilated patient system (100), the ventilated patient system including a respiratory subsystem of the patient and inspiratory and expiratory accessories (632, 634, 640, 642), the patient model estimator adapted to perform quantification of respiratory muscle effort of the patient by

i) establishing a respiratory predictive model of the ventilated patient system based on an equation of motion and one or more periodic or semi-periodic functions that approximate clinically-observed, patient-generated muscle pressures,

**characterized in that**:

the patient model estimator is further configured to perform quantification of the respiratory muscle effort of the patient by:

ii) determining an instantaneous leak flow value for the ventilated patient system, including a leak flow value for constant size leak opening and a pressure-dependent varying-size leak opening, and

iii) based on the received one or more measured or estimated characteristics and the instantaneous leak flow value, solving the respiratory predictive model to extract a respiratory muscle effort value; and

the ventilator system further comprises a controller (110, 650) operable to control various aspects of delivery of the flow of gas to the patient based on the respiratory muscle effort value or one or more other respiratory parameters derived based on the respiratory muscle effort value.

14. The ventilator system of claim 13, wherein the one or more periodic or semi-periodic functions that approximate clinically-observed, patient-generated muscle pressures include a periodic or semi-periodic function that approximates clinically-observed, inspiratory muscle pressures and the respiratory muscle effort value comprises an estimate of inspiratory muscle effort generated by the patient,
and/or
wherein said solving the respiratory predictive model to extract a respiratory muscle effort value includes optimizing derived parameters of the equation of motion,
and/or
wherein the patient model estimator is further adapted to perform continuous online quantification of respiratory muscle effort of the patient.

15. The ventilator system of claim 14, wherein the periodic function for inspiration is expressed as:

$$P_{musi}(t) = -P_{\max}\,(1 - \frac{t}{t_v})\sin(\frac{\pi t}{t_v})$$

where,
$P_{\max}$ represents a maximum inspiratory muscle pressure;
$t_v$ represents duration of inspiration; and
t represents an elapsed breath time varying between 0 and a total sum of inspiration and expiration periods.

16. The ventilator system of claim 13, wherein the one or more periodic or semi-periodic functions that approximate clinically-observed, patient-generated muscle pressures include a periodic or semi-periodic function that approximates clinically-observed, expiratory muscle pressures and the respiratory muscle effort value comprises an estimate of expiratory muscle effort generated by the patient.

17. The ventilator system of claim 16, wherein a periodic function for an expiratory phase of respiration is expressed as:

$$P_{muse}(t) = P_{\max}\,(\frac{t}{t_v})\sin(\frac{\pi(t - t_v)}{t_{tot} - t_v})$$

where,
$P_{\max}$ represents a maximum expiratory muscle pressure;
$t_v$ represents duration of expiration;
ttot represents a total sum of inspiration and expiration periods;
t represents an elapsed breath time varying between 0 and $t_{tot}$.

18. The ventilator system of claim 15, wherein the respiratory predictive model is assumed to be valid for a plurality of breath cycles of the patient and the method further comprises periodically reestablishing, updating or optimizing the respiratory predictive model at predetermined temporal windows during breath cycles of the patient.

19. The ventilator system of claim 18, wherein said solving the respiratory predictive model to extract a respiratory muscle effort value comprises solving the respiratory predictive model during a breath cycle of the plurality of breath cycles subsequent to establishment of the respiratory predictive model and correcting the respiratory muscle effort value to account for time delays introduced by measurement and indirect indication of muscular activity by surrogate phenomena.

**20.** The ventilator system of claim 19 and claim 17, wherein said correcting the respiratory muscle effort value to account for time delays involves application of a single-pole dynamic expressed as:

$$P_{mus, deliver}(s) = \frac{We^{-s\tau}}{s + z} P_{mus}(s)$$

where,

W represents a scaling factor incorporating a magnitude ratio of actual to delivered muscle pressure;

r represents a delay time constant; and

z represents the single pole; and

$$P_{mus}(s) = (\pi) \frac{\frac{P_{max}}{t_v}(s - \frac{\pi}{t_v})^2}{\left[s^2 + \left(\frac{\pi}{t_v}\right)^2\right]^2}$$

for inspiration

and,

$$P_{mus}(s) = (\pi \frac{P_{max}}{t_v(t_{tot} - t_v)}) \frac{t_v\left[s^2 + \left(\frac{\pi}{t_{tot} - t_v}\right)^2\right] + 2s}{\left[s^2 + \left(\frac{\pi}{t_{tot} - t_v}\right)^2\right]^2}$$

for exhalation.

**21.** The ventilator system of claim 13 wherein the pressure-dependent varying-size leak opening is an elastic leak orifice component and the constant size leak opening is an inelastic leak orifice component.

**22.** The ventilator system of claim 21, wherein:

an instantaneous leak flow for the elastic leak orifice is determined to be proportional to $(\Delta P)^{3/2}$; and
an instantaneous leak flow for the inelastic leak orifice is determined to be proportional to $(\Delta P)^{1/2}$;
wherein $\Delta P$ is equal to a pressure differential across the leak orifices.

**Patentansprüche**

**1.** Verfahren, umfassend:

Empfangen, Messen oder Schätzen einer oder mehrerer Eigenschaften eines Patientenbeatmungsgeräts, die Werte interessierender Parametern darstellen, die mit statischen oder dynamischen Eigenschaften oder Attributen eines Patientenbeatmungssystems (100) verknüpft sind, wobei das Patientenbeatmungssystem (100) ein Beatmungssubsystem eines Patienten (140, 624) und ein Beatmungssystem (620) aufweist, das dem Patienten einen Gasstrom zuführt;
Durchführen einer Quantifizierung der Atemmuskelanstrengung des Patienten durch

i) Erstellen eine Atmungsprognosemodells des Patientenbeatmungssystems basierend auf einer Bewegungsgleichung und einer oder mehreren Funktionen, die die klinisch beobachteten, vom Patienten erzeugten Muskeldrucke näherungsweise bestimmen,

**dadurch gekennzeichnet, dass**:

das Durchführen der Quantifizierung der Atemmuskelanstrengung des Patienten ferner umfasst:

ii) Bestimmen eines momentanen Leckage-Flusswerts für das Patientenbeatmungssystem mit einem Leckage-Flusswert für eine Leckageöffnung konstanter Größe und für eine druckabhängige Leckageöffnung variabler Größe, und

iii) Berechnen des Beatmungsprognosemodells zum Extrahieren eines geschätzten Werts der physiologischen Atemmuskelanstrengung basierend auf der einen oder den mehreren Eigenschaften des Patientenbeatmungsgeräts und dem momentanen Leckage-Flusswert; und

wobei das Verfahren weiterhin aufweist den Schritt eines Konfigurierens und Betreibens des Beatmungssystems basierend auf dem Schätzwert der physiologischen Atemmuskelanstrengung oder auf anderen Parametern, die zu Überwachungszwecken oder zum Zwecke der Atemzufuhr daraus abgeleitet wurden.

2. Verfahren nach Anspruch 1, wobei die eine Funktion oder die mehreren Funktionen periodische oder halbperiodische Funktionen umfassen.

3. Verfahren nach Anspruch 2, wobei die periodischen oder halbperiodischen Funktionen konstante Amplituden aufweisen,
und/oder
wobei die eine oder die mehreren periodischen oder halbperiodischen Funktionen Amplituden aufweisen, die im Zeitverlauf variieren.

4. Verfahren nach Anspruch 1, wobei die eine oder die mehreren Funktionen, die die klinisch beobachteten, vom Patienten erzeugten Muskeldrucke näherungsweise bestimmen, eine inspiratorische periodische Funktion für eine Einatmungsphase der Atmung aufweisen, die die klinisch beobachteten Muskeldrucke beim Einatmen näherungsweise bestimmt, und der Schätzwert der physiologischen Atemmuskelanstrengung eine Schätzung der vom Patienten erzeugten Muskelanstrengung beim Einatmen umfasst.

5. Verfahren nach Anspruch 4, wobei die inspiratorische periodische Funktion dargestellt wird durch:

$$P_{musi}(t) = -P_{max}\left(1 - \frac{t}{t_v}\right)\sin\left(\frac{\pi t}{t_v}\right)$$

wobei
$P_{max}$ einen maximalen Muskeldruck beim Einatmen darstellt, der ein konstanter Parameter oder ein im Zeitverlauf variierender Parameter sein kann;
$t_v$ die Dauer der Einatmung darstellt; und
t eine verstrichene Atemzeit darstellt, die zwischen 0 und der Gesamtsumme der Einatmungs- und Ausatmungszeitspannen variiert.

6. Verfahren nach Anspruch 1, wobei die eine oder die mehreren Funktionen, die die klinisch beobachteten, vom Patienten erzeugten Muskeldrucke näherungsweise bestimmen, eine exspiratorische periodische Funktion für eine Ausatmungsphase der Atmung aufweisen, die die klinisch beobachteten Muskeldrucke beim Ausatmen näherungsweise bestimmt, und der Schätzwert der physiologischen Atemmuskelanstrengung eine Schätzung der vom Patienten erzeugten Muskelanstrengung beim Ausatmen umfasst.

7. Verfahren nach Anspruch 6, wobei die exspiratorische periodische Funktion dargestellt wird durch:

$$P_{muse}(t) = P_{max}\left(\frac{t}{t_v}\right)\sin(\frac{\pi(t - t_v)}{t_{tot} - t_v})$$

wobei
$P_{max}$ einen maximalen Muskeldruck beim Ausatmen darstellt, der ein konstanter Parameter oder ein im Zeitverlauf variierender Parameter sein kann;
$t_v$ die Dauer der Ausatmung darstellt;
$t_{tot}$ eine Gesamtsumme von Einatmungs- und Ausatmungszeitspannen darstellt; und
t eine verstrichene Atemzeit darstellt, die zwischen 0 und $t_{tot}$ variiert.

8. Verfahren nach Anspruch 5, wobei das Atmungsprognosemodell für eine Vielzahl von Atemzyklen des Patienten als gültig angenommen wird, und das Verfahren weiterhin das erneute Erstellen, Aktualisieren oder Optimieren des Atmungsprognosemodells in periodischen Abständen in bestimmten Zeitfenstern während der Atmungszyklen des Patienten umfasst.

9. Verfahren nach Anspruch 8, wobei das Berechnen des Atmungsprognosemodells zum Extrahieren eines Schätzwerts der physiologischen Atemmuskelanstrengung das Berechnen eines Atemprognosemodells während eines Atemzyklus der Vielzahl der Atemzyklen nach dem Erstellen des Atemprognosemodells und das Ausgleichen des Schätzwerts der physiologischen Atemmuskelanstrengung für durch ein Messsystem und die indirekte Angabe der Muskelaktivität durch Ersatzphänome bedingte Zeitverzögerungen umfasst.

10. Verfahren nach Anspruch 9 und Anspruch 7, wobei das Ausgleichen des Schätzwerts der physiologischen Atemmuskelanstrengung für Zeitverzögerungen die Anwendung einer Ein-Pol-Dynamik beinhaltet, die dargestellt ist durch:

$$P_{mus,deliver(s)} = \frac{We^{-s\tau}}{s + z} P_{mus(s)}$$

wobei
W einen Skalierungsfaktor darstellt, der ein Größenverhältnis des tatsächlichen zu dem bereitgestellten Muskeldruck beinhaltet;
T eine Zeitverzögerungskonstante darstellt; und
z den einzelnen Pol darstellt; und

$$P_{mus(s)} = (\pi)\frac{\frac{P_{max}}{t_v}(s - \frac{\pi}{t_v})^2}{\left[s^2 + \left(\frac{\pi}{t_v}\right)^2\right]^2};$$

für die Einatmung und

$$P_{mus(s)} = (\pi \frac{P_{max}}{t_v(t_{tot} - t_v)}) \frac{t_v\left[s^2 + \left(\frac{\pi}{t_{tot} - t_v}\right)^2\right] + 2s}{\left[s^2 + \left(\frac{\pi}{t_{tot} - t_v}\right)^2\right]^2} \; ;$$

für die Ausatmung gilt.

11. Verfahren nach Anspruch 1, wobei das Berechnen des Atmungsprognosemodells zum Extrahieren des Werts der Atemmuskelanstrengung die Optimierung abgeleiteter Parameter der Bewegungsgleichung auf laufender Basis aufweist, um die Dynamik des Patientenbeatmungssystems zu optimieren.

12. Verfahren nach Anspruch 11, wobei das Atmungsverhalten des Patienten zur Dynamik gehört.

13. Beatmungssystem (100), mit:

einer Beatmungsgerät-Patienten-Schnittstelle (628), über die einem Patienten (140, 624) ein Gasstrom zugeführt wird;
einem Patientenmodellschätzsystem (150, 350), das betreibbar ist, um Messungen oder Schätzungen von einer oder mehreren Patienten-Beatmungsgerät-Eigenschaften des Patientenbeatmungssystems (100) zu erhalten, wobei das Patientenbeatmungssystem ein Atmungssubsystem des Patienten und Einatmungs- und Ausatmungszubehör (632, 634, 640, 642) aufweist, wobei das Patientenmodellschätzsystem eine Quantifizierung der Atemmuskelanstrengung des Patienten durchzuführen vermag durch

i) Erstellen eines Atmungsprognosemodells des Patientenbeatmungssystems basierend auf einer Bewegungsgleichung und einer oder mehreren periodischen oder halbperiodischen Funktionen, die die klinisch beobachteten, vom Patienten erzeugten Muskeldrucke näherungsweise bestimmen,

**dadurch gekennzeichnet, dass**:

das Patientenmodellschätzsystem ferner dazu ausgebildet ist, eine Quantifizierung der Atemmuskelanstrengung des Patienten durchzuführen durch:

ii) Bestimmen eines momentanen Leckage-Flusswerts für das Patientenbeatmungssystem mit einem Leckage-Flusswert für eine Leckageöffnung konstanter Größe und für eine druckabhängige Leckageöffnung variabler Größe, und
iii) Berechnen des Beatmungsprognosemodells zum Extrahieren eines Werts der Atemmuskelanstrengung basierend auf der einen oder den mehreren empfangenen gemessenen oder geschätzten Eigenschaften und dem momentanen Leckage-Flusswert; und

wobei das Beatmungssystem weiterhin eine Steuereinheit (110, 650) umfasst, die betreibbar ist, um verschiedene Aspekte der Gasstromzufuhr zum Patienten basierend auf dem Wert der Atemmuskelanstrengung oder auf einem oder mehreren weiteren Atmungsparametern zu steuern, die basierend auf dem Wert der Atemmuskelanstrengung abgeleitet werden.

14. Beatmungssystem nach Anspruch 13, wobei die eine oder die mehreren periodischen oder halbperiodischen Funktionen, die die klinisch beobachteten, vom Patienten erzeugten Muskeldrucke näherungsweise bestimmen, eine periodische oder halbperiodische Funktion aufweisen, die die klinisch beobachteten Muskeldrucke beim Einatmen näherungsweise bestimmt, und der Wert der Atemmuskelanstrengung eine Schätzung der vom Patienten erzeugten Muskelanstrengung beim Einatmen umfasst,
und/oder
wobei das Berechnen des Atmungsprognosemodells zum Extrahieren eines Werts der Atemmuskelanstrengung das Optimieren der abgeleiteten Parameter der Bewegungsgleichung aufweist,
und/oder
wobei das Patientenmodellschätzsystem ferner dazu ausgebildet ist, eine fortlaufende Online-Quantifizierung der Atemmuskelanstrengung des Patienten durchzuführen.

15. Beatmungssystem nach Anspruch 14, wobei die inspiratorische periodische Funktion ausgedrückt ist als:

$$P_{musi}(t) = -P_{max}\left(1 - \frac{t}{t_v}\right)\sin(\frac{\pi t}{t_v})$$

wobei
$P_{max}$ den maximalen Muskeldruck beim Einatmen darstellt;
$t_v$ die Dauer der Einatmung darstellt; und
t eine verstrichene Atemzeit darstellt, die zwischen 0 und der Gesamtsumme der Einatmungs- und Ausatmungszeitspannen variiert.

16. Beatmungssystem nach Anspruch 13, wobei die eine oder die mehreren periodischen oder halbperiodischen Funktionen, die die klinisch beobachteten, vom Patienten erzeugten Muskeldrucke näherungsweise bestimmen, eine periodische oder halbperiodische Funktion aufweisen, die die klinisch beobachteten Muskeldrucke beim Ausatmen näherungsweise bestimmt, und der Wert der Atemmuskelanstrengung eine Schätzung der vom Patienten erzeugten Muskelanstrengung beim Ausatmen umfasst.

17. Beatmungssystem nach Anspruch 16, wobei eine periodische Funktion für eine Ausatmungsphase der Atmung ausgedrückt ist als:

$$P_{muse}(t) = P_{max}\left(\frac{t}{t_v}\right)\sin(\frac{\pi(t - t_v)}{t_{tot} - t_v})$$

wobei
$P_{max}$ den maximalen Muskeldruck beim Ausatmen darstellt;
$t_v$ die Dauer der Ausatmung darstellt; und
$t_{tot}$ eine Gesamtsumme der Einatmungs- und Ausatmungszeitspannen darstellt;;
t eine verstrichene Atmungszeit darstellt, die zwischen 0 und $t_{tot}$ variiert.

18. Beatmungssystem nach Anspruch 15, wobei das Atmungsprognosemodell für eine Vielzahl von Atemzyklen des Patienten als gültig angenommen wird, und das Verfahren ferner ein erneutes Erstellen, Aktualisieren oder Optimieren des Atmungsprognosemodells in periodischen Abständen in bestimmten Zeitfenstern während der Atmungszyklen des Patienten umfasst.

19. Beatmungssystem nach Anspruch 18, wobei das Berechnen des Atmungsprognosemodells zum Extrahieren eines Werts der Atemmuskelanstrengung das Berechnen des Atemprognosemodells während eines Atemzyklus der Vielzahl der Atemzyklen nach dem Erstellen des Atemprognosemodells und das Korrigieren des Werts für die Atemmuskelanstrengung umfasst, um die durch das Messen und die indirekte Angabe der Muskelaktivität durch Ersatzphänome bedingten Zeitverzögerungen zu berücksichtigen.

20. Beatmungssystem nach Anspruch 17 und 19, wobei die Korrektur des Werts der Atemmuskelanstrengung zur Berücksichtigung der Zeitverzögerungen die Anwendung einer Ein-Pol-Dynamik beinhaltet, die ausgedrückt ist als:

$$P_{mus,deliver(s)} = \frac{We^{-s\tau}}{s + z}P_{mus(s)}$$

wobei

W einen Skalierungsfaktor darstellt, der ein Größenverhältnis des tatsächlichen zu dem bereitgestellten Muskeldruck aufweist;
T eine Zeitverzögerungskonstante darstellt; und
z den einzelnen Pol darstellt; und

$$P_{mus(s)} = (\pi)\frac{\frac{P_{\max}}{t_v}(s - \frac{\pi}{t_v})^2}{\left[s^2 + \left(\frac{\pi}{t_v}\right)^2\right]^2};$$

für die Einatmung
und

$$P_{mus(s)} = (\pi \frac{P_{\max}}{t_v(t_{tot} - t_v)})\frac{t_v\left[s^2 + \left(\frac{\pi}{t_{tot} - t_v}\right)^2\right] + 2s}{\left[s^2 + \left(\frac{\pi}{t_{tot} - t_v}\right)^2\right]^2};$$

für die Ausatmung gilt.

21. Beatmungssystem nach Anspruch 13, wobei die druckabhängige Leckageöffnung variabler Größe eine elastische Leckageöffnungskomponente und die Leckageöffnung konstanter Größe eine unelastische Leckageöffnungskomponente ist.

22. Beatmungssystem nach Anspruch 21, wobei:

ein momentaner Leckagefluss für die elastische Leckageöffnung als proportional zu $(\Delta P)^{3/2}$ festgelegt ist; und
ein momentaner Leckagefluss für die unelastische Leckageöffnung als proportional zu $(\Delta P)^{1/2}$ festgelegt ist; und wobei $\Delta P$ gleich einer Druckdifferenz über den Leckageöffnungen ist.

**Revendications**

1. Procédé comprenant:

la réception, la mesure ou l'estimation d'une ou plusieurs caractéristiques patient-ventilateur représentant des valeurs de paramètres d'intérêt associés avec des propriétés ou des attributs statiques ou dynamiques d'un système (100) de patient ventilé, le système (100) de patient ventilé incluant un sous-système respiratoire d'un patient (140, 624) et un système de ventilation (620), qui délivre un flux de gaz au patient;
l'exécution d'une quantification d'effort musculaire respiratoire du patient par i) l'établissement d'un modèle prédictif respiratoire du système de patient ventilé sur la base d'une équation de mouvement et d'une ou plusieurs fonctions qui approche(nt) des pressions musculaires générées par le patient cliniquement observées, **caractérisé en ce que**:

l'exécution d'une quantification d'effort musculaire respiratoire du patient comprend en outre:

ii) la détermination d'une valeur instantanée de flux de fuite pour le système de patient ventilé, incluant une valeur de flux de fuite pour une ouverture de fuite de taille constante et une ouverture de fuite de taille variable en fonction de la pression,

et

iii) sur la base de l'une ou des plusieurs caractéristiques patient-ventilateur et de la valeur instantanée de flux de fuite, la résolution du modèle prédictif respiratoire pour extraire une valeur d'effort musculaire respiratoire physiologique estimée; et

le procédé comprend en outre l'étape de configuration et d'utilisation du système de ventilation sur la base de la valeur d'effort musculaire respiratoire physiologique estimée ou d'autres paramètres dérivés de celle-ci à des fins de surveillance ou de délivrance de respiration.

2. Procédé selon la revendication 1, dans lequel l'une ou les plusieurs fonctions comprend/comprennent des fonctions périodiques ou semi-périodiques.

3. Procédé selon la revendication 2, dans lequel les fonctions périodiques ou semi-périodiques ont des amplitudes constantes, et/ou
dans lequel l'une ou les plusieurs fonctions périodiques ou semi-périodiques ont des amplitudes variant avec le temps.

4. Procédé selon la revendication 1, dans lequel l'une ou les plusieurs fonctions qui approche(nt) des pressions musculaires générées par le patient cliniquement observées, inclut/incluent une fonction d'inspiration périodique pour une phase d'inspiration de la respiration qui approche des pressions musculaires à l'inspiration cliniquement observées et la valeur d'effort musculaire respiratoire physiologique estimée comprend une estimation de l'effort musculaire à l'inspiration généré par le patient.

5. Procédé selon la revendication 4, dans lequel la fonction d'inspiration périodique est exprimée comme:

$$P_{musi}(t) = -P_{max}\left(1 - \frac{t}{t_v}\right)\sin\left(\frac{\pi t}{t_v}\right)$$

où
$P_{max}$ représente une pression musculaire maximum à l'inspiration, qui peut être une constante ou un paramètre variant avec le temps;
$t_v$ représente une durée de l'inspiration; et
$t$ représente une durée de respiration écoulée variant entre 0 et une somme totale de périodes d'inspiration et d'expiration.

6. Procédé selon la revendication 1, dans lequel l'une ou les plusieurs fonctions qui approche(nt) des pressions musculaires générées par le patient cliniquement observées, inclut/incluent une fonction d'expiration périodique pour une phase d'expiration de la respiration qui approche des pressions musculaires à l'expiration cliniquement observées et la valeur d'effort musculaire respiratoire physiologique estimée comprend une estimation de l'effort musculaire à l'expiration généré par le patient.

7. Procédé selon la revendication 6, dans lequel la fonction d'expiration périodique est exprimée comme:

$$P_{muse}(t) = P_{max}\left(\frac{t}{t_v}\right)\sin\left(\frac{\pi(t - t_v)}{t_{tot} - t_v}\right)$$

où
$P_{max}$ représente une pression musculaire maximum à l'expiration, qui peut être une constante ou un paramètre variant avec le temps;
$t_v$ représente une durée de l'expiration;
$t_{tot}$ représente une somme totale de périodes d'inspiration et d'expiration; et t représente une durée de respiration écoulée variant entre 0 et $t_{tot}$.

8. Procédé selon la revendication 5, dans lequel le modèle prédictif respiratoire est supposé être valide pour une pluralité de cycles respiratoires du patient et le procédé comprend en outre le rétablissement, la mise à jour ou l'optimisation périodique du modèle prédictif respiratoire selon des fenêtre temporelles prédéterminées pendant des cycles respiratoires du patient.

9.  Procédé selon la revendication 8, dans lequel ladite résolution du modèle prédictif respiratoire pour extraire une valeur d'effort musculaire respiratoire physiologique estimée comprend la résolution du modèle prédictif respiratoire pendant un cycle respiratoire parmi la pluralité de cycles respiratoires faisant suite à l'établissement du modèle prédictif respiratoire et la compensation de la valeur d'effort musculaire respiratoire physiologique estimée pour des retards temporels introduits par un système de mesure et une indication indirecte d'activité musculaire par des phénomènes auxiliaires.

10. Procédé selon la revendication 9 et la revendication 7, dans lequel ladite compensation de la valeur d'effort musculaire respiratoire physiologique estimée pour des retards temporels implique l'application d'une dynamique unipolaire exprimée comme:

$$P_{mus,\,deliver}(s) = \frac{We^{-s\tau}}{s+z}\,P_{mus(s)}$$

où

W représente un facteur de mise à l'échelle incorporant un rapport de grandeur de la pression musculaire réelle sur délivrée;

$\tau$ représente une constante de retard temporel; et

z représente le pôle unique; et

$$P_{mus}(s) = (\pi)\frac{\dfrac{P_{max}}{t_v}\left(s - \dfrac{\pi}{t_v}\right)^2}{\left[s^2 + \left(\dfrac{\pi}{t_v}\right)^2\right]^2}$$

pour l'inspiration et

$$P_{mus}(s) = \left(\pi\frac{P_{max}}{t_v(t_{tot}-t_v)}\right)\frac{t_v\left[s^2 + \left(\dfrac{\pi}{t_{tot}-t_v}\right)^2\right]+2s}{\left[s^2 + \left(\dfrac{\pi}{t_{tot}-t_v}\right)^2\right]^2}$$

pour l'expiration.

11. Procédé selon la revendication 1, dans lequel ladite résolution du modèle prédictif respiratoire pour extraire une valeur d'effort musculaire respiratoire inclut l'optimisation de paramètres dérivés de l'équation de mouvement sur une base continue pour s'accorder sur la dynamique du système de patient ventilé.

12. Procédé selon la revendication 11, dans lequel la dynamique inclut un comportement de respiration du patient.

**13.** Système de ventilateur (100) comprenant:

une interface ventilateur-patient (628) par l'intermédiaire de laquelle un flux de gaz est délivré à un patient (140, 624);

un estimateur de modèle de patient (150, 350) utilisable pour recevoir des mesures ou des estimations d'une ou plusieurs caractéristiques patient-ventilateur du système (100) de patient ventilé, le système de patient ventilé incluant un sous-système respiratoire du patient et des accessoires d'inspiration et d'expiration (632, 634, 640, 642), l'estimateur de modèle de patient adapté à exécuter une quantification d'effort musculaire respiratoire du patient par

i) l'établissement d'un modèle prédictif respiratoire du système de patient ventilé sur la base d'une équation de mouvement et d'une ou plusieurs fonctions périodiques ou semi-périodiques qui approche(nt) des pressions musculaires générées par le patient cliniquement observées,

**caractérisé en ce que**:

l'estimateur de modèle de patient est en outre configuré pour exécuter une quantification de l'effort musculaire respiratoire du patient par:

ii) la détermination d'une valeur instantanée de flux de fuite pour le système de patient ventilé, incluant une valeur de flux de fuite pour une ouverture de fuite de taille constante et une ouverture de fuite de taille variable en fonction de la pression,
et

iii) sur la base de l'une ou des plusieurs caractéristiques mesurées ou estimées reçues et de la valeur instantanée de flux de fuite, la résolution du modèle prédictif respiratoire pour extraire une valeur d'effort musculaire respiratoire; et

le système de ventilateur comprend en outre un contrôleur (110, 650) utilisable pour commander divers aspects de délivrance du flux de gaz au patient sur la base de la valeur d'effort musculaire respiratoire ou d'un ou plusieurs autres paramètres respiratoires dérivés sur la base de la valeur d'effort musculaire respiratoire.

**14.** Système de ventilateur selon la revendication 13, dans lequel l'une ou les plusieurs fonctions périodiques ou semi-périodiques qui approche(nt) des pressions musculaires générées par le patient cliniquement observées, inclut/incluent une fonction périodique ou semi-périodique qui approche des pressions musculaires à l'inspiration cliniquement observées et la valeur d'effort musculaire respiratoire comprend une estimation de l'effort musculaire à l'inspiration généré par le patient, et/ou

dans lequel ladite résolution du modèle prédictif respiratoire pour extraire une valeur d'effort musculaire respiratoire inclut l'optimisation de paramètres dérivés de l'équation de mouvement, et/ou

dans lequel l'estimateur de modèle de patient est en outre adapté à exécuter une quantification continue en ligne de l'effort musculaire respiratoire du patient.

**15.** Système de ventilateur selon la revendication 14, dans lequel la fonction périodique pour l'inspiration est exprimée comme:

$$P_{musi}(t) = -P_{max}\left(1 - \frac{t}{t_v}\right)\sin\left(\frac{\pi t}{t_v}\right)$$

où

$P_{max}$ représente une pression musculaire maximum à l'inspiration;

$t_v$ représente une durée de l'inspiration; et

$t$ représente une durée de respiration écoulée variant entre 0 et une somme totale de périodes d'inspiration et d'expiration.

**16.** Système de ventilateur selon la revendication 13, dans lequel l'une ou les plusieurs fonctions périodiques ou semi-périodiques qui approche(nt) des pressions musculaires générées par le patient cliniquement observées, inclut/incluent une fonction périodique ou semi-périodique qui approche des pressions musculaires à l'expiration cliniquement observées et la valeur d'effort musculaire respiratoire comprend une estimation de l'effort musculaire à l'expiration généré par le patient.

**17.** Système de ventilateur selon la revendication 16, dans lequel une fonction périodique pour une phase d'expiration de respiration est exprimée comme:

$$P_{muse}(t) = P_{max}\left(\frac{t}{t_v}\right)\sin\left(\frac{\pi(t-t_v)}{t_{tot}-t_v}\right)$$

où

$P_{max}$ représente une pression musculaire maximum à l'expiration;

$t_v$ représente une durée de l'expiration;

$t_{tot}$ représente une somme totale de périodes d'inspiration et d'expiration;

$t$ représente une durée de respiration écoulée variant entre 0 et $t_{tot}$.

**18.** Système de ventilateur selon la revendication 15, dans lequel le modèle prédictif respiratoire est supposé être valide pour une pluralité de cycles respiratoires du patient et le procédé comprend en outre le rétablissement, la mise à jour ou l'optimisation périodique du modèle prédictif respiratoire selon des fenêtre temporelles prédéterminées pendant des cycles respiratoires du patient.

**19.** Système de ventilateur selon la revendication 18, dans lequel ladite résolution du modèle prédictif respiratoire pour extraire une valeur d'effort musculaire respiratoire comprend la résolution du modèle prédictif respiratoire pendant un cycle respiratoire parmi la pluralité de cycles respiratoires faisant suite à l'établissement du modèle prédictif respiratoire et la correction de la valeur d'effort musculaire respiratoire pour prendre en compte des retards temporels introduits par une mesure et une indication indirecte d'activité musculaire par des phénomènes auxiliaires.

**20.** Système de ventilateur selon la revendication 19 et la revendication 17, dans lequel ladite correction de la valeur d'effort musculaire respiratoire pour prendre en compte des retards temporels implique l'application d'une dynamique unipolaire exprimée comme:

$$P_{mus,deliver}(s) = \frac{We^{-s\tau}}{s+z}P_{mus(s)}$$

où

W représente un facteur de mise à l'échelle incorporant un rapport de grandeur de la pression musculaire réelle sur délivrée;

$\tau$ représente une constante de retard temporel; et

z représente le pôle unique; et

$$P_{mus}(s) = (\pi)\frac{\frac{P_{max}}{t_v}\left(s-\frac{\pi}{t_v}\right)^2}{\left[s^2+\left(\frac{\pi}{t_v}\right)^2\right]^2}$$

pour l'inspiration
et

$$P_{mus}(s) = (\pi \frac{P_{max}}{t_v (t_{tot} - t_v)}) \frac{t_v \left[ s^2 + \left( \frac{\pi}{t_{tot} - t_v} \right)^2 \right] + 2s}{\left[ s^2 + \left( \frac{\pi}{t_{tot} - t_v} \right)^2 \right]^2}$$

pour l'expiration.

21. Système de ventilateur selon la revendication 13, dans lequel l'ouverture de fuite de taille variable en fonction de la pression est un composant d'orifice de fuite élastique et l'ouverture de fuite de taille constante est un composant d'orifice de fuite non élastique.

22. Système de ventilateur selon la revendication 21, dans lequel:

un flux de fuite instantané pour l'orifice de fuite élastique est déterminé être proportionnel à $(\Delta P)^{3/2}$; et
un flux de fuite instantané pour l'orifice de fuite non élastique est déterminé être proportionnel à $(\Delta P)^{1/2}$;
dans lesquelles $\Delta P$ est égal à un différentiel de pression entre les orifices de fuite.

FIG. 1

FIG. 2

Estimated Physiologic
Patient Respiratory Effort
330

Patient-Ventilator
Characteristics
335

Controller
Interface
325

Operational
Instructions
320

Processor
305

Memory
310

Sensor Measurements
315

Patient Model Estimator 350

**FIG. 3**

EP 2 348 995 B1

Ventilator Control Processing

410

Parameter
measurement /
estimation period has
elapsed?

N

420

Measure or estimate those system parameters of a ventilated
patient system that can be measured or estimated

430

Perform online patient model estimation to determine a patient
respiratory effort value and/or other desired parameters

440

Configure the ventilation system based on the patient
respiratory effort value and/or other extracted parameters

Y

**FIG. 4**

```
              ┌─────────────────────────────┐
              │   Online Quantification of  │
              │   Respiratory Muscle Effort │
              │         Processing          │
              └──────────────┬──────────────┘
                             │
                             ▼
   510                  ◇ Predefined temporal ◇
                          window during breath ──────────┐
                               cycle?                     │
                             │                            │
                             │ Y                          │
                             ▼                            │
   520  ┌─────────────────────────────────────────────┐  │
        │ Establish, reestablish, update or optimize a│  │ N
        │ respiratory predictive model of the         │  │
        │ ventilated patient system                   │  │
        └──────────────────┬──────────────────────────┘  │
                           │◄────────────────────────────┘
   530                     ▼
        ┌─────────────────────────────────────────────┐
        │ Determine the instantaneous leak flow for   │
        │ the ventilated patient system               │
        └──────────────────┬──────────────────────────┘
                           │
   540                     ▼
  ┌───────────────────────────────────────────────────────┐
  │ Solve the current respiratory predictive model based  │
  │ on the available parameters and based on the current  │
  │ time offset into the current breath to extract a      │
  │ respiratory muscle effort value and/or other desired  │
  │ parameters                                            │
  └──────────────────────┬────────────────────────────────┘
                         │
   550                   ▼
        ┌─────────────────────────────────────────┐
        │ Compensate parameter(s) for time delays │
        └──────────────────┬──────────────────────┘
                           │
                           ▼
                  ┌─────────────────┐
                  │       End       │
                  └─────────────────┘
```

# FIG. 5

FIG. 6

FIG. 7

Ventilator Detects Trigger          Ventilator Detects Trigger

FIG. 8A

Airway Pressure

Time

Patient's Effort Starts          Patient's Effort Starts

Leak
Condition

Flow

Leak
Condition

FIG. 8B

No Leak

No Leak

Time

928

928

961

FIG. 9A          FIG. 9B

```
              ┌─────────────┐
              │    Start    │
              └─────────────┘
                     │
                     ▼                      ┌─ 1012
              ┌──────────────────────────┐
              │  Establish Baseline Control │
              └──────────────────────────┘
                     │
                     ▼                      ┌─ 1014
              ┌──────────────────────────┐
              │ Implement Ventilator Feedback │
              │          Control          │
              └──────────────────────────┘
                     │
                     ▼                      ┌─ 1016
      1018 ┌────────────────────────────────────────┐ ┌─ 1020
           │           Leak Compensation            │
           │  ┌──────────────┐  ┌──────────────────┐ │
           │  │ Rigid Orifice │  │ Elastic Compensation │ │
           │  │ Compensation  │  │                  │ │
           │  └──────────────┘  └──────────────────┘ │
           └────────────────────────────────────────┘
                     │                      ┌─ 1022
                     ▼
              ┌──────────────────────────┐
              │  Adjust Appropriate Control │
              │  Commands and Correct/    │
              │  Compensate Applicable    │
              │      Measurements         │
              └──────────────────────────┘
                     │
                     ▼
              ┌─────────────┐
              │    Return   │
              └─────────────┘
```

# FIG. 10

**EP 2 348 995 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2002028460 A

- US 61041070 A

### Non-patent literature cited in the description

- **JAFARI, M.** Robust Feedback Design for Proportional Assist Ventilation-System Dynamics and Problem Definition. *Decision and Control, 2005 and "2005 European Control Conference, CDC-E CC '05, 44th IEEE Conference,* 12 December 2005, ISBN 987-0-7803-9567-1, 4839-4844